(19) Europäisches Patentamt / European Patent Office / Office européen des brevets

(11) **EP 4 144 762 A1**

(12) **EUROPEAN PATENT APPLICATION**
published in accordance with Art. 153(4) EPC

(43) Date of publication:
**08.03.2023 Bulletin 2023/10**

(21) Application number: **21797335.3**

(22) Date of filing: **27.04.2021**

(51) International Patent Classification (IPC):
**C07K 17/08** (2006.01)     **C12N 7/02** (2006.01)

(52) Cooperative Patent Classification (CPC):
**C07K 17/08; C12N 7/02**

(86) International application number:
**PCT/JP2021/016772**

(87) International publication number:
**WO 2021/221050 (04.11.2021 Gazette 2021/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.04.2020 JP 2020078298**

(71) Applicant: **Kaneka Corporation
Osaka-shi, Osaka 530-8288 (JP)**

(72) Inventors:
• **SUEOKA, Takuma
  Takasago-shi, Hyogo 676-8688 (JP)**
• **NISHIHACHIJO, Masakatsu
  Takasago-shi, Hyogo 676-8688 (JP)**
• **YAURA, Hisako
  Takasago-shi, Hyogo 676-8688 (JP)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **STRUCTURE, METHOD FOR PRODUCING SAME, ADSORBENT IN WHICH SAME IS USED, AND METHOD FOR PURIFYING BIOPARTICLES**

(57) A structure includes a water-insoluble fiber, and a low-molecular-weight antibody connected to the water-insoluble fiber. A method of producing a structure includes connecting a water-insoluble fiber and a low-molecular-weight antibody to each other.

EP 4 144 762 A1

**Description**

Technical Field

The present invention relates to a structure, a method of producing the structure, an adsorbent that uses the structure, and a method of purifying biological particles.

Background Art

Biological particles, such as viruses, virus-like particles, virus vectors, or the like, are very important molecules in medical fields, such as gene therapy, vaccines, laboratory diagnostics, hemocatharsis, or the like. Also, removal of such biological particles is very important in ensuring prevention and prophylaxis of infections and virological safety of pharmaceuticals.

In recent years, these medical fields have been attracting particular attention. Thus, there has been a strong demand for not only a technique to efficiently remove biological particles, but also a method to efficiently recover biological particles from biological samples, such as cell culture solution, blood, or the like.

A method that is generally used to purify viruses to high purity is one that first removes solid matter, such as cells, cell debris, and the like, through filtration from culture solution of transformed cells or chorioallantoic liquid of chick embryo in which viruses are produced, and then purifies the cultures or chorioallantoic liquid through, for example, chromatography or ultracentrifugation.

Affinity chromatography is a method of separating target substances and other impurities by using carriers obtained by immobilizing, to bead base materials, ligands such as antibodies or the like that specifically bind to target molecules. From the viewpoints of safety and productivity, examples using low-molecular-weight antibodies as ligands are known (e.g., PTL 1). In addition, also in traditional virus purification methods, a method to use bead carriers to which low-molecular-weight antibodies are immobilized (e.g., NPL 1) and commercially available products such as (CAPTO (registered trademark), obtained from AVB GE, POROS (registered trademark) CaptureSelect (registered trademark) AAVX, obtained from Thermo Scientific), or the like are known.

In traditional chromatography using bead carriers, however, the bead carriers are expensive and are difficult to prepare, and the pore diameters of the bead base materials are small, which causes a disadvantage in efficiently recovering biological particles, such as viruses or the like.

As described above, any of a structure and a method of producing the structure, an adsorbent using the structure, and a method of purifying biological particles that are inexpensive, have a high recovery rate of biological particles in an elution step, and a low residual rate of biological particles in a washing step, is not known at all, and there is a strong demand for provision of them.

Citation List

Patent Literature

PTL 1: International Publication No. WO01/44301

Non-Patent Literature

NPL 1: Mol. Ther. Methods Clin. Dev. 2018 9:33

Summary of Invention

Technical Problem

The present invention solves the above various problems in the art and achieves the following object. That is, an object of the present invention is to provide a structure and a method of producing the structure, an adsorbent using the structure, and a method of purifying biological particles that are inexpensive and have a high recovery rate of biological particles in an elution step and a low residual rate of biological particles in a washing step. In addition, in removal of the biological particles, the present invention provides a method that is also effective as effective removing means for these.

Solution to Problem

The present inventors conducted intensive studies to achieve the above object and have obtained the following findings. Specifically, a structure including a water-insoluble fiber and a low-molecular-weight antibody connected to the water-insoluble fiber can provide a structure that is inexpensive and has a high recovery rate of biological particles in an elution step and a low residual rate of biological particles in a washing step. Use of a method of producing a structure including connecting a water-insoluble fiber and a low-molecular-weight antibody to each other can provide a method of producing a structure that is inexpensive and has a high recovery rate of biological particles in an elution step and a low residual rate of biological particles in a washing step.

The present invention is based on the above findings obtained by the present inventors. Means for solving the above problems are as follows.

<1> A structure, including:

a water-insoluble fiber; and
a low-molecular-weight antibody connected to the water-insoluble fiber.

<2> A method of producing a structure, the method including:
connecting a water-insoluble fiber and a low-molecular-weight antibody to each other.
<3> An adsorbent, including:
the structure according to <1> above.
<4> A method of purifying biological particles, the method including:
using the structure according to <1> above or the adsorbent according to <3> above.

Advantageous Effects of Invention

According to the present invention, it is possible to solve the above various problems in the art and achieve the above object, and to provide a structure and a method of producing the structure, an adsorbent using the structure, and a method of purifying biological particles that are inexpensive and have a high recovery rate of biological particles in an elution step and a low residual rate of biological particles in a washing step.

Brief Description of Drawings

FIG. 1 is a graph that indicates a correlation between an average pore diameter calculated with a perm porometer and a reciprocal of a square root of an air passage time.
FIG. 2 is a graph that indicates a chromatogram of AAV adsorption and desorption by carrier 7.
FIG. 3 is a graph that indicates a chromatogram of AAV adsorption and desorption by POROS (registered trademark) CaptureSelect (registered trademark) AAVX.

Description of Embodiments

(Structure)

The structure includes a water-insoluble fiber, and a low-molecular-weight antibody connected to the water-insoluble fiber. The structure may further include other elements.

That is, in the structure, the water-insoluble fiber and the low-molecular-weight antibody may be directly connected to each other, or the water-insoluble fiber and the low-molecular-weight antibody may be connected to each other via other elements.

-Water-insoluble fiber-

The water-insoluble fiber can be used as a base material of the structure.

The lower limit of the thickness of the water-insoluble fiber is not particularly limited and may be appropriately selected depending on the intended purpose. From the viewpoint of stability in production, the lower limit thereof is preferably 0.08 mm or higher, more preferably 0.10 mm or higher, and still more preferably 0.12 mm or higher.

The upper limit of the thickness of the water-insoluble fiber is not particularly limited and may be appropriately selected depending on the intended purpose. From the viewpoint of uniformity in areal weight, the upper limit thereof is preferably 0.50 mm or lower, more preferably 0.40 mm or lower, and still more preferably 0.30 mm or lower.

The lower limit of the areal weight of the water-insoluble fiber is not particularly limited and may be appropriately selected depending on the intended purpose. From the viewpoint of stability in production, the lower limit thereof is preferably 5 g/m² or higher and more preferably 10 g/m² or higher.

The upper limit of the areal weight of the water-insoluble fiber is not particularly limited and may be appropriately selected depending on the intended purpose. From the viewpoint of uniformity in the water-insoluble fiber, the upper limit thereof is preferably 100 g/m² or lower, more preferably 90 g/m² or lower, still more preferably 80 g/m² or lower, and particularly preferably 70 g/m² or lower.

The lower limit of the bulk density of the water-insoluble fiber is not particularly limited and may be appropriately selected depending on the intended purpose. From the viewpoint of smaller changes in the structure of the water-insoluble fiber after insertion into a device, the upper limit thereof is preferably 50 kg/m³ or higher, more preferably 60 kg/m³ or higher, and still more preferably 70 kg/m³ or higher.

The upper limit of the bulk density of the water-insoluble fiber is not particularly limited and may be appropriately selected depending on the intended purpose. From the viewpoint of liquid-passage performance, the upper limit thereof is preferably 400 kg/m³ or lower, more preferably 350 kg/m³ or lower, and still more preferably 300 kg/m³ or lower.

Note that, the bulk density refers to a value obtained by measuring the weight of the water-insoluble fiber per 1 m³ of the water-insoluble fiber.

The shape of the water-insoluble fiber is not particularly limited and may be appropriately selected depending on the intended purpose. Examples of the shape thereof include a circle, a rectangle, a triangle, and a bale shape.

The surface of the water-insoluble fiber may be modified through graft polymerization, polymer coating, a chemical treatment with, for example, an alkali or acid, or a plasma treatment.

The graft polymerization is not particularly limited and may be appropriately selected depending on the intended purpose. Examples of the graft polymerization include graft polymerization method involving irradiation with electron beams.

The graft polymerization involving irradiation with electron beams is classified into a so-called pre-irradiation method and a so-called simultaneous irradiation method. In the pre-irradiation method, the water-insoluble fiber is irradiated with electron beams in advance to generate radicals, followed by application of a radical-polymerizable compound to the water-insoluble fiber containing the generated radicals, to promote polymerization of a graft polymerizable compound by the subsequent post-polymerization. In the simultaneous irradiation method, a radical-polymerizable compound is applied to the water-insoluble fiber, followed by the irradiation with electron beams to generate radicals, to promote polymerization of a graft polymerizable compound by the subsequent post-polymerization.

In the present invention, any of the pre-irradiation method and the simultaneous irradiation method can be used.

In either of the pre-irradiation method and the simultaneous irradiation method, it is preferable to laminate a film on the surface of the water-insoluble fiber for a duration of from the application of the radical-polymerizable compound to the water-insoluble fiber, to completion of the post-polymerization. This prevents vaporization of the radical-polymerizable compound to initiate graft polymerization uniformly. In addition, the blocking of the air prevents deactivation of radicals by oxygen in the air. In the simultaneous irradiation method, the surface of the water-insoluble fiber is sealed with a film also at the time of the irradiation with electron beams. In this state, the water-insoluble fiber and the radical-polymerizable compound are blocked from oxygen in the air. Thus, oxidation of the water-insoluble fiber due to oxygen in the air does not readily occur.

The film to be used is a polymer film having a thickness of from 0.01 to 0.20 mm, and the thickness of the film is appropriately determined depending on the penetrating power of the electron beams to be used.

The material of the film is not particularly limited and may be appropriately selected depending on the intended purpose. Examples of the material thereof include polyester-based materials, such as polyethylene terephthalate and the like, and polyolefin-based materials. Of these, polyethylene terephthalate is preferable. Particularly in the simultaneous irradiation method, it is suitable to use a polyethylene terephthalate film having a low generation yield of polymer radicals by the irradiation with electron beams and having low oxygen permeability.

Note that, the polyester-based material means material of polyester and a derivative thereof, and the polyolefin-based material means material of polyolefin and a derivative thereof.

In the pre-irradiation method, first, the water-insoluble fiber is irradiated with electron beams to generate radicals that will induce polymer reaction (e.g., polymer radicals). The temperature of the atmosphere at the time of the irradiation with electron beams may be usual room temperature although the radical generation yield is higher at lower temperatures.

Irradiation conditions for the electron beams in the pre-irradiation method are not particularly limited and may be appropriately selected depending on the intended purpose. In a state where the oxygen concentration of the atmosphere for the irradiation is set to 300 ppm or lower, the acceleration voltage is preferably from 100 to 2000 kilovolts (hereinafter abbreviated as "kV"), more preferably from 120 to 300 kV, and the current is from 1 to 100 mA. Within these ranges, the irradiation conditions are appropriately determined depending on, for example, the thickness of the water-insoluble fiber and the target graft rate.

The irradiation dose of the electron beams may be appropriately determined in consideration of the target graft rate and degradation in physical properties of the water-insoluble fiber by the irradiation. Typically, the irradiation dose of about from 10 to 300 kilograys (hereinafter abbreviated as "kGy") is appropriate, with the irradiation dose of from 10 to 200 kGy being preferable. At the irradiation dose of less than 10 kGy, a necessary number of radicals for a sufficient level of graft polymerization are not generated. At the irradiation dose of higher than 300 kGy, even when the water-insoluble fiber is formed of a polymer material having resistance to radiation, the main chain of the polymer material is cut to cause degradation in physical properties, which is not preferable.

Note that, the atmosphere for the irradiation in the pre-irradiation method is preferably an inert gas atmosphere such as nitrogen gas or the like but may be air. In air, however, the water-insoluble fiber may be oxidized by oxygen contained therein.

Then, a radical-polymerizable compound is applied to the water-insoluble fiber after the irradiation with electron beams. For example, the water-insoluble fiber is retained for a predetermined duration by being immersed in a tank of a radical-polymerizable compound solution from which dissolved oxygen has been removed through passage of nitrogen gas or by immersing and passing the tank of the radical-polymerizable compound solution, to apply a sufficient amount of the radical-polymerizable compound to the water-insoluble fiber.

Note that, the term "immersion" or "immersing" in the present invention means that the water-insoluble fiber contacts the radical-polymerizable compound solution. Therefore, as a method of applying the radical-polymerizable compound to the water-insoluble fiber, any of various coating methods can be used. Of these, impregnation coating, comma direct coating, comma reverse coating, kiss coating, gravure coating, etc. are preferable because these can achieve efficient coating.

The water-insoluble fiber to which the radical-polymerizable compound has been applied is taken out from the solution tank. At this time, it is preferable to laminate a film on the surface of the water-insoluble fiber. For example, when the water-insoluble fiber is in a sheet or fiber shape, the water-insoluble fiber, to which the radical-polymerizable compound solution has been applied, is held between two films and closely attached to the films. By closely attaching the films to the water-insoluble fiber to which the radical-polymerizable compound solution has been applied, it is possible to control the application rate of the radical-polymerizable compound solution to be constant, and uniformly apply the radical-polymerizable compound to the water-insoluble fiber. Also, by allowing the water-insoluble fiber and the radical-polymerizable compound to react with each other in a space hermetically sealed by laminating the films, the graft reaction is more promoted.

Note that, the term "laminate" in the present invention means that the water-insoluble fiber is allowed to contact the films.

When the water-insoluble fiber taken out from the solution tank is retained in a post-polymerization tank of a predetermined temperature for a predetermined duration, the graft polymerization of the radical-polymerizable compound is promoted (post-polymerization). The temperature for the post-polymerization is from 0°C to 130°C and more preferably from 40°C to 70°C. This promotes the graft polymerization reaction between the water-insoluble fiber and the radical-polymerizable compound. After that, the resulting product is washed and dried to be able to produce a grafted fiber. Note that, the atmosphere for the post-polymerization is preferably an inert gas atmosphere such as nitrogen gas or the like but when the post-polymerization is performed in a state of laminating the films, the atmosphere for the post-polymerization may be air.

In the simultaneous irradiation method, the water-insoluble fiber is retained for a predetermined duration by being immersed in a tank of a radical-polymerizable compound solution from which dissolved oxygen has been removed through passage of nitrogen gas or by immersing and passing the tank of the radical-polymerizable compound solution, to apply a sufficient amount of the radical-polymerizable compound to the water-insoluble fiber. After that, the water-insoluble fiber is taken out from the solution tank, followed by the irradiation with electron beams. Preferably, the surface of the water-insoluble fiber is laminated with films when the water-insoluble fiber is taken out from the solution tank, and in this state, the water-insoluble fiber is irradiated with electron beams.

The acceleration voltage in the simultaneous irradiation method may be appropriately determined depending on the kind of the polymer material, the total thickness of the water-insoluble fiber, to which the radical-polymerizable compound solution has been applied, and the films laminated, and the target graft rate. Usually, the acceleration voltage is appropriately from about 100 to about 2000 kV. Also, the irradiation dose of the electron beams may be similar to that in the pre-irradiation method. The atmosphere at the time of irradiation with electron beams is preferably an inert gas atmosphere, such as nitrogen, helium, or the like. However, when the surface of the water-insoluble fiber is laminated with the films, the atmosphere for the irradiation does not influence the graft polymerization. Thus, considering cost saving, irradiation in air is appropriate.

Similar to the pre-irradiation method, the irradiation of the water-insoluble fiber with the electron beams in the simultaneous irradiation method is followed by the post-polymerization of the radical-polymerizable compound. After that, the resulting product is washed and dried to be able to produce a grafted fiber. In addition, also in the simultaneous irradiation method, the atmosphere for the post-polymerization is preferably an inert gas atmosphere such as nitrogen gas or the like, but when the post-polymerization is performed in a state of laminating the films, the atmosphere for the post-polymerization may be air.

Also, the radical-polymerizable compound used in the present invention is may be a compound that forms a bond with a polymer radical generated in the water-insoluble fiber by the irradiation with electron beams. Specific examples of the radical-polymerizable compound include, but are not limited to: unsaturated compounds each having an acidic group, such as acrylic acid, methacrylic acid, itaconic acid, methacrylsulfonic acid, styrenesulfonic acid, and the like, and esters thereof; unsaturated carboxylic acid amides such as acrylamides, methacrylamides, and the like; unsaturated compounds each having a glycidyl group or a hydroxy group at the end thereof; unsaturated organophosphates such as vinyl phosphonate and the like; and methacrylic acid esters, fluoroacrylates, and acrylonitrile each having basic group, such as quaternary ammonium salts, tertiary ammonium salts, and the like. These may be used alone or in combination. When two or more radical-polymerizable compounds are used, a composite grafted fiber is produced, in which the graft chain is formed of a copolymer of at least two radical-polymerizable compounds.

Of these radical-polymerizable compounds, acrylic-based monomers are preferably used in the present invention from the viewpoint of graft rate. Further, from the viewpoint of reactivity with a ligand having an amino group, a hydroxy group, a thiol group, etc., acrylic-based monomers each having a carboxy group or an epoxy group at the end of a molecule thereof are preferable, and at least one selected from the group consisting of acrylic acid, methacrylic acid, and glycidyl methacrylate (hereinafter abbreviated as "GMA") is more preferable.

Note that, the acrylic-based monomer means monomer of acrylic and a derivative thereof.

The above radical-polymerizable compound may be in the form of a dilute solution containing, as a solvent, water or an organic solvent such as lower alcohol or the like, or a mixed solution thereof. The concentration of the radical-polymerizable compound in this dilute solution varies with the intended graft rate, but the dilute solution can be prepared at the concentration of the radical-polymerizable compound of from 1 to 70% by volume. Also, when a radical-polymerizable compound that readily forms a homopolymer is used, a metal salt of copper or iron may be added to the dilute solution of the radical-polymerizable compound, to prevent formation of a homopolymer.

The lower limit of the concentration of the radical-polymerizable compound in the solution is not particularly limited and may be appropriately selected depending on the intended purpose. The lower limit thereof is preferably 1% by weight or higher, more preferably 2.5% by weight or higher, still more preferably 5% by weight or higher, and particularly preferably 10% by weight or higher.

The upper limit of the concentration of the radical-polymerizable compound in the solution is not particularly limited and may be appropriately selected depending on the intended purpose. The upper limit thereof is preferably 70% by mass or lower, more preferably 60% by weight or lower, still more preferably 50% by weight or lower, and particularly preferably 40% by weight or lower.

Also, when the radical-polymerizable compound solution contains a solvent, the graft rate increases. In this case, the concentration of an emulsifier in the solvent is preferably adjusted to be in the range of from 0.1 to 5% by weight.

The emulsifier is not particularly limited and may be appropriately selected depending on the intended purpose. For example, polysorbates are preferable. Examples of the polysorbates include POLYSOBATEs 20, 60, 65, and 80. Of these, POLYSOBATE 20, which has high hydrophilicity, is more preferable.

Note that, it is desirable to sparge the radical-polymerizable compound solution with inert gas such as nitrogen gas or the like to remove dissolved oxygen therefrom in advance.

The graft rate in the graft polymerization reaction is not particularly limited and may be appropriately selected depending on the intended purpose. The graft rate is preferably 50% or higher.

The "graft rate" in the present invention is a value calculated as follows from the dry weight of the water-insoluble fiber before the graft reaction (W1) and the dry weight of the grafted fiber after the graft reaction (W2):

$$\texttt{Graft rate = [(W2-W1)/W1]×100 (\%).}$$

The material of the water-insoluble fiber is not particularly limited and may be appropriately selected depending on the intended purpose. Examples of the material thereof include polyolefin-based materials, polypropylene, polypropylene maleic anhydride, modified polypropylene, polyethylene, cellulose, regenerated cellulose, cellulose acetate, cellulose diacetate, cellulose triacetate, ethyl cellulose, cellulose acetate, polyethylene terephthalate (PET), polybutylene terephthalate (PBT), acrylic resins, polycarbonate, polyester-based materials, polyacrylonitrile, polyamide, polystyrene, brominated polystyrene, polyalkyl (meth)acrylate, polyvinyl chloride, polychloroprene, polyurethane, polyvinyl alcohol, polyvinyl acetate, polysulfone, polyethersulfone, polybutadiene, a butadiene-acrylonitrile copolymer, a styrene-butadiene copolymer, an ethylene-vinyl alcohol copolymer, aramid, glass, nylon, and rayon. These may be used alone or in combination. Of these, from the viewpoint of exhibiting good reactivity in the graft polymerization by the irradiation with electron beams, polyolefin-based materials or cellulose-based materials are preferable, polyolefin-based materials are more preferable, and polypropylene is still more preferable.

Note that, the polyolefin-based material means material of polyolefin and a derivative thereof, the polyester-based material means material of polyester and a derivative thereof, and the cellulose-based material means material of cellulose and a derivative thereof.

The lower limit of the average fiber diameter of the water-insoluble fiber is not particularly limited and may be appropriately selected depending on the intended purpose. From the viewpoint of exhibiting good tensile strength or of productivity, the lower limit thereof is preferably 0.3 µm or higher, more preferably 0.4 µm or higher, and still more preferably 0.5 µm or higher.

The upper limit of the average fiber diameter of the water-insoluble fiber is not particularly limited and may be appropriately selected depending on the intended purpose. From the viewpoint of exhibiting superior purification, the upper limit thereof is preferably 15 µm or lower, more preferably 10 µm or lower, still more preferably 5 µm or lower, and particularly preferably 3 µm or lower. The fiber having the average fiber diameter exceeding 15 µm is not preferable because of poor purification.

The lower limit of the average pore diameter of the water-insoluble fiber is not particularly limited and may be appropriately selected depending on the intended purpose. From the viewpoint of exhibiting good performance in liquid passage or of productivity, the lower limit thereof is preferably 1.0 µm or higher, more preferably higher than 1.0 µm, still more preferably 1.5 µm or higher, even more preferably 2.0 µm or higher, particularly preferably 2.5 µm or higher, and most preferably 3.0 µm or higher.

The upper limit of the average pore diameter of the water-insoluble fiber is not particularly limited and may be appropriately selected depending on the intended purpose. From the viewpoint of exhibiting high performance in purification, the upper limit thereof is preferably 50 µm or lower, more preferably 40 µm or lower, still more preferably 35 µm or lower, and particularly preferably 30 µm or lower.

The average pore diameter of the water-insoluble fiber is measured in the following manner.

Specifically, for the average pore diameter of the water-insoluble fiber, the mean flow pore size measured with a perm porometer (obtained from Porous Materials Inc. (PMI), Capillary Flow Porometer CFP-1200AEXLCSHJ) is defined as the average pore diameter.

For measurement and calculation of the average pore diameter, measurement software (Autofilling Perm-Porometer v6.73.90) and analysis software (CAPREP v6.71.51) are used.

The water-insoluble fiber to be evaluated is punched out into a circle having a diameter of 26 mm, followed by immersion in Galwick (registered trademark in the US) penetration liquid (obtained from PMI, with a surface tension of 15.9 Dynes/cm). The resulting product is placed in a desiccator, from which air is removed by reducing the pressure with a vacuum pump for about 1 minute, and is charged with the penetration liquid. This water-insoluble fiber is set on a sample stage (Φ26) attached to the device. The flow rate of air to the sample impregnated with the penetration liquid is gradually increased to 200000 cc/m. After that, the flow rate of air is gradually decreased until the pressure reaches 0 kPa in a dry state. The pressure in this process is plotted, and the average flow rate diameter (average pore diameter) is calculated by the bubble point method. Conditions for the measurement are set as follows.

Maxflow 200000 (cc/m)
Bublflow 2.00 (cc/m)
F/PT 200 (oldbubltime)
Minbpress 0 (kPa)
Zerotime 1.0 (sec)
V2incr 5 (cts*3)
Preginc 0.3
Pulsedelay 2
Maxpres 1378.9466 (kPa)
Pulsewidth 0.2 (sec)
Mineqtime 30 (sec)
Presslew 10 (cts*3)
Flowslew 50 (cts*3)
Equiter 5 (0.1 sec)
Aveiter 20 (0.1 sec)
Maxpdif 0.69 (kPa)
Maxfdif 50 (cc/m)

The Gurley permeability of the water-insoluble fiber (the time (sec) required for the total amount of air of 300 mL to pass through the water-insoluble fiber) is not particularly limited and may be appropriately selected depending on the intended purpose. The Gurley permeability of the water-insoluble fiber is preferably 60 seconds or less, more preferably 30 seconds or less, still more preferably 10 seconds or less, even more preferably 8 seconds or less, particularly preferably 5 seconds or less, and most preferably 2.5 seconds or less.

The Gurley permeability is a value obtained according to JIS L1096:2010 (8.26.2 B method (Gurley type method)). In this method, the water-insoluble fiber is set on a circular air-passage surface having an area of 642 mm$^2$. A cylinder having a height of 254 mm, an outer diameter of 76.2 mm, an inner diameter of 74 mm, and a mass of 567 g is used to pass 300 mL of air through the water-insoluble fiber. The time required for passage of the total amount of air of 300 mL (air passage time: sec) is measured. At this time, 1 to 30 sheets of the water-insoluble fiber are stacked on top of one another depending on the pore diameters. Measurement is performed three times for each level to calculate the average value per sheet.

The water-insoluble fiber is not particularly limited and may be appropriately selected depending on the intended purpose. The water-insoluble fiber may be a nonwoven fabric or may be a woven fabric or a knitted fabric. However, from the viewpoint of simplification of a production step, a nonwoven fabric is preferable.

A method of producing the nonwoven fabric is not particularly limited and may be appropriately selected depending on the intended purpose. Examples of the method include a wet method, a dry method, a melt-blow method, an electrospinning method, a flash spinning method, a papermaking method, a spunbond method, a thermal bond method, a chemical bond method, a needle punch method, a spunlace method (a hydroentanglement method), a stitch bond method, and a steam jet method. Of these, from the viewpoint of obtaining an ultrafine fiber, a melt-blow method, an electrospinning method, a flash spinning method, a papermaking method, etc. are preferable.

The melt-blow method is not particularly limited and may be appropriately selected depending on the intended purpose. Examples of the melt-blow method include a method of blowing a thermoplastic resin, which has been melted with an extruder, from a melt-blow die into a filament by the action of a high-temperature, high-speed air flow, to accumulate a fibrously stretched resin on a conveyor, where the resulting fibers are entangled and fuse and adhere to each other, to produce a nonwoven fabric of self-adherent ultrafine fibers without any binder. At this time, by adjusting, for example, a resin viscosity, a melting temperature, a discharge amount, a hot-air temperature, a wind pressure, and DCD (the distance from the surface of a spinneret to the conveyor), it is possible to control the fiber diameter, areal weight, fiber orientation, and fiber dispersibility of the nonwoven fabric. Further, it is possible to control the thickness of the nonwoven fabric and the average pore diameter through, for example, a heat-press process or a tenter process.

-Low-molecular-weight antibody-

"Antibody" is a common name that is given by focusing on the functions of an immunoglobulin. The immunoglobulin is a glycoprotein produced by B cells of lymphocytes, and has the function of recognizing a molecule such as a certain protein or the like to bind to the molecule. The immunoglobulin has the function of specifically binding to this certain molecule (antigen) and the function of detoxifying and removing a factor having the antigen together with other biomolecules and cells.

All of the immunoglobulins fundamentally have the same molecular structure. Each of them has a fundamental structure of four chains in the shape of the letter "Y" (i.e., two polypeptide chains for light chains and two polypeptide chains for heavy chains). The light chains (L chains) are two kinds, a λ chain and a κ chain, and each immunoglobulin has either of them. The heavy chains (H chains) are five kinds of different structures; i.e., a γ chain, a μ chain, an α chain, a δ chain, an ε chain. Based on the difference in the heavy chain, the kind (isotype) of the immunoglobulin changes. Immunoglobulin G (hereinafter may be abbreviated as "IgG") is a monomeric immunoglobulin and is composed of two heavy chains (γ chains) and two light chains, and has two antigen-binding sites.

A portion of the antibody corresponding to the lower-half vertical bar of the letter "Y" is called a Fc region, and a portion of the antibody corresponding to the letter "V" of the upper half thereof is called a Fab region. The Fc region has the effector function of triggering reaction after the antibody binds to an antigen, and the Fab region has the function of binding to an antigen. The Fab region and the Fc region of the heavy chain are linked via a hinge region. A protease papain contained in papaya cleaves this hinge region to divide the antibody into two Fab regions (fragments) and one Fc region. Portions (domains) near the leading ends of the letter "Y" in the Fab regions have variable amino acid sequences so as to be able to bind to various antigens. Thus, these portions are called a variable region (V region). The variable region of the light chain is called a VL region and the variable region of the heavy chain is called a VH region. The Fab region other than the V region, and the Fc region are regions having relatively small changes and are called a constant region (C region). The constant region of the light chain is called a CL region and the constant region of the heavy chain is called a CH region. The CH region is further divided into three, CH1 to CH3. The Fab region of the heavy chain is composed of the VH region and the CH1 and the Fc region of the heavy chain is composed of the CH2 and the CH3. The hinge region is located between the CH1 and the CH2. Among antibodies derived from camelids and antibodies derived from fishes such as a shark or the like, there are heavy-chain antibodies, which are composed of only heavy chains without any light chains. The heavy-chain antibodies derived from camelids are distinguished from a usual IgG antibody (IgG1) having light chains, and are called IgG2 and IgG3. Meanwhile, the heavy-chain antibodies derived from fishes are called an IgNAR (new antigen receptor).

The low-molecular-weight antibody of the present invention is an antibody fragment lacking of part of the whole antibody (e.g., the whole IgG). The low-molecular-weight antibody is not particularly limited as long as the antibody fragment has an ability to bind to an antigen.

The low-molecular-weight antibody of the present invention is not particularly limited and may be appropriately selected depending on the intended purpose. Preferably, the low-molecular-weight antibody lacks the CH2 domain and the CH3 domain.

The low-molecular-weight antibody of the present invention preferably includes the heavy-chain variable region (VH) or the light-chain variable region (VL), or both. The amino acid sequence of the VH or the VL may include addition, deletion, and/or substitution. Moreover, as long as the low-molecular-weight antibody binds to an antigen, part of the VH or part of the VL, or parts of both can be lacking.

The low-molecular-weight antibody of the present invention is not particularly limited and may be appropriately selected depending on the intended purpose. Examples thereof include low-molecular-weight antibodies including a variable region of a heavy-chain antibody derived from a camelid (VHH), a variable region of a heavy-chain antibody derived from fishes (V-NAR), Fab, Fab', F(ab')$_2$, a single chain antibody (scFv), a diabody, a triabody, and a minibody. Of these, from the viewpoints of stability and productivity, the low-molecular-weight antibodies including a variable region of a heavy-chain antibody derived from a camelid (VHH) and a single chain antibody (scFv) are preferable.

The variable region of the heavy-chain antibody derived from the camelid (VHH) is not particularly limited and may be appropriately selected depending on the intended purpose. Examples thereof include a product obtained through purification of a serum of a camelid immunized with the adsorption target, a product obtained by allowing a host cell to express a VHH gene, and a product obtained through chemical synthesis based on the amino acid sequence.

Also, the VHH may be a commercially available product.

The camelid is not particularly limited and may be appropriately selected depending on the intended purpose. Examples of the camelid include a two-humped camel, a one-humped camel, llama, alpaca, vicuna, and guanaco.

The host cell that is allowed to express the VHH gene is not particularly limited and may be appropriately selected depending on the intended purpose. Examples of the host cell include bacteria such as *Escherichia coli* (*E. coli*) and the like, fungi such as a yeast and the like, animal cells, and plant cells.

A method of immunizing the camelid with the adsorption target is not particularly limited and may be appropriately selected depending on the intended purpose. Examples of the method include the method described in International Publication No. WO2020/067418.

A method of allowing the host cell to express and produce the VHH gene is not particularly limited and may be appropriately selected depending on the intended purpose. Examples of the method include the method described in International Publication No. WO2020/067418 and the method described in Japanese Patent Application Laid-Open (JP-A) No. 2015-119637.

The commercially available product of the VHH is not particularly limited and may be appropriately selected depending on the intended purpose. Examples of the commercially available product include CaptureSelect (registered trademark) Biotin Anti-HSA Conjugate, CaptureSelect (registered trademark) Biotin Anti-IgG-Fc (Human) Conjugate, and CaptureSelect (registered trademark) Biotin Anti-AAVX Conjugate, all of which are obtained from Thermo Scientific.

The single chain antibody (scFv) is obtained by linking the VH and the VL of an antibody. In the scFv, the VH and the VL are linked via a linker, preferably a peptide linker (Proc. Natl. Acad. Sci. U.S.A 1988 85:5879). The peptide linker is not particularly limited. For example, any single chain peptide of from about 3 to about 25 residues can be used as the linker.

The scFv is not particularly limited and may be appropriately selected depending on the intended purpose. Examples of the scFv include a product obtained by allowing a host cell to express the scFv, a product obtained through chemical synthesis based on the amino acid sequence, and a product obtained by linking, via a linker, a heavy-chain variable region and a light-chain variable region of an antibody having a known sequence.

Also, the scFv may be a commercially available product.

The host cell that is allowed to express the scFv gene is not particularly limited and may be appropriately selected depending on the intended purpose. Examples of the host cell include bacteria such as *Escherichia coli* and the like, fungi such as a yeast and the like, animal cells, and plant cells.

The low-molecular-weight antibody of the present invention preferably includes a single domain antibody.

The single domain antibody is not particularly limited and may be appropriately selected depending on the intended purpose. The single domain antibody preferably includes the variable region of the heavy-chain antibody derived from the camelid (VHH).

The low-molecular-weight antibody of the present invention may be chimerized or humanized.

The molecular weight of the low-molecular-weight antibody is not particularly limited and may be appropriately selected depending on the intended purpose. The molecular weight thereof is preferably 130,000 or lower, more preferably 100,000 or lower, still more preferably 50,000 or lower, particularly preferably 30,000 or lower, and most preferably 20,000 or lower.

The adsorption target of the low-molecular-weight antibody is not particularly limited and may be appropriately selected depending on the intended purpose. Examples of the adsorption target include viruses, virus-like particles, virus vectors, nucleic acids, antibodies, enzymes, hormones, and composites and metabolites thereof. Of these, viruses, virus-like particles, and virus vectors are preferable.

The virus-like particle is all or part of the virus outer envelope proteins that mainly form a capsid. The virus-like particle contains no nucleic acid and has no infectivity. Meanwhile, the virus-like particle triggers immune reaction and thus can be used as an effective ingredient of a vaccine.

The viruses, virus-like particles, and virus vectors are not particularly limited and may be appropriately selected depending on the intended purpose. Examples thereof include: non-enveloped viruses, such as adeno-associated virus (AAV), adenovirus, enterovirus, parvovirus, papovavirus, human papillomavirus, rotavirus, coxsackievirus, sapovirus, norovirus, poliovirus, echovirus, hepatitis A virus, hepatitis E virus, rhinovirus, astrovirus, circovirus, simian virus, and the like; enveloped viruses, such as retrovirus, lentivirus, Sendai virus, herpesviruses such as herpes simplex virus and the like, vaccinia virus, measles virus, baculovirus, influenza virus, leukemia virus, Sindbis virus, and the like; and capsids of these viruses or virus vectors containing these genes. Of these, adeno-associated virus or a capsid of adeno-associated virus, or a vector containing an adeno-associated virus gene is preferable.

The adeno-associated virus is a virus containing linear single-stranded DNA in a capsid thereof and belonging to the Family *Parvoviridae*. As serotypes of the adeno-associated virus, for example, Type 1 AAV (AAV1), Type 2 AAV (AAV2), Type 3 AAV (AAV3), Type 4 AAV (AAV4), Type 5 AAV (AAV5), Type 6 AAV (AAV6), Type 7 AAV (AAV7), Type 8 AAV (AAV8), Type 9 AAV (AAV9), and Type 10 AAV (AAV10) are known.

When the adsorption target of the low-molecular-weight antibody is the adeno-associated virus, the known VHH described in International Publication No. WO2018/104528 can be used as the low-molecular-weight antibody.

Also, commercially available products such as CaptureSelect (registered trademark) Biotin Anti-AAVX Conjugate of Thermo Scientific or the like can be used.

In addition, an IgG antibody such as Intact Particles [A20] antibody of GeneTex or the like can be fragmented and used.

When the low-molecular-weight antibody such as the scFv or the like is produced from an antibody having a known sequence, it is possible to use sequences that can be obtained from a public database, such as sequences that are registered in a protein databank or the like; e.g., a heavy chain sequence (PDB:3J1S_H) and a light chain sequence (PDB:3J1S_L) of an antibody that binds to AAV2.

--AAV-binding capacity--

The AAV-binding capacity of the structure is not particularly limited and may be appropriately selected depending on the intended purpose. The AAV-binding capacity of the structure is preferably $1.0 \times 10^{12}$ vg/mL-column or more, more preferably $5.0 \times 10^{12}$ vg/mL-column or more, still more preferably $1.0 \times 10^{13}$ vg/mL-column or more, and particularly preferably $1.5 \times 10^{13}$ vg/mL-column or more. Note that, a method of calculating the binding capacity is a method based on the total adsorption amount calculated by subtracting, from a load amount, an elution volume to a flow-through fraction, or a method based on the total amount recovered in an elution step after adsorption. In the present invention, the binding capacity is calculated based on the total recovery amount in the latter method.

The AAV-binding capacity of the structure is measured by the following method.

An AAV crude liquid is purified through affinity chromatography using the structure.

The following Liquids A to F are prepared, and were filtered through a 0.2-μm filter before use.

Liquid A: Tris-HCl (20mmol/L), sodium chloride (500 mmol/L), pH 8.0
Liquid B: citric acid (100 mmol/L), sodium chloride (500 mmol/L), pH 2.1
Liquid C: NaOH (10 mmol/L)
Liquid D: phosphoric acid (120 mmol/L), acetic acid (167 mmol/L), benzyl alcohol (2.2%v/v)
Liquid E: 20% ethanol
Liquid F: a solution prepared by diluting the AAV crude liquid with Liquid A and adjusted to pH 8.0 (about $2 \times 10^{13}$ vg)

A column is connected to AKTA Avant 25. The flow rate of this system is set to 0.2 mL/min. The column is washed with pure water and equilibrated with Liquid A. Next, Liquid F is passed therethrough to retain the AAV to the column carrier, followed by washing with Liquid A and eluting the AAV with Liquid B. After completion of elution, the column is washed with Liquid A, then Liquid C, then Liquid A, and then Liquid D. The AAV amount contained in each fraction is measured through qPCR, to calculate the binding capacity from the AAV amount contained in the elution step.

--Ligand density--

The ligand density (the low-molecular-weight antibody density) of the structure is not particularly limited and may be appropriately selected depending on the intended purpose. The ligand density of the structure is preferably 0.01 mg/mL-column or more but 100 mg/mL-column or less, more preferably 0.1 mg/mL-column or more but 50 mg/mL-column or less, and still more preferably 1 mg/mL-column or more but 10 mg/mL-column or less.

The ligand density is measured in the following manner.

Specifically, a filtrate, obtained when immobilizing the ligand to the water-insoluble fiber, is recovered, the absorbance of the filtrate is then measured, and the ligand density of the ligand immobilized to the water-insoluble fiber is then calculated.

-Other elements-

The other elements are not particularly limited and may be appropriately selected depending on the intended purpose. Examples of the other elements include a spacer.

--Spacer--

The structure including the spacer is not particularly limited and may be appropriately selected depending on the intended purpose. Examples thereof include a structure where the low-molecular-weight antibody is connected via the spacer to the water-insoluble fiber.

A functional group contained in the spacer is not particularly limited and may be appropriately selected depending on the intended purpose. Examples of the functional group include an amino group, a hydroxy group, an epoxy group, and a carboxy group. Of these, an epoxy group is preferable from the viewpoints of stability, reactivity, and easiness to connect to the ligand.

The spacer is not particularly limited and may be appropriately selected depending on the intended purpose. Examples thereof include spacers including a polymer, a monomer, a dimer, a trimer, and a tetramer. Of these, the spacer including a polymer is preferable. The polymer may be a copolymer.

The polymer is not particularly limited and may be appropriately selected depending on the intended purpose. Examples thereof include polymers including a hydrophilic polymer and a hydrophobic polymer. Of these, the polymer including a hydrophilic polymer is preferable from the viewpoints of the ability to be handled in an aqueous medium and preventing non-specific hydrophobic actions between the protein and the spacer.

The hydrophilic polymer is not particularly limited and may be appropriately selected depending on the intended purpose. Examples thereof include hydrophilic polymers including polyamines and polysaccharides.

The polyamines are not particularly limited and may be appropriately selected depending on the intended purpose. Examples of the polyamines include polyethyleneimine, polyallylamine, polyvinylamine, and polylysine, diamines, such as ethylenediamine, 1,2-propanediamine, 1,6-hexamethylenediamine, piperazine, 2,5-dimethylpiperazine, isophoronediamine, 4,4'-dicyclohexylmethanediamine, 1,4-cyclohexanediamine, and the like; polyamines, such as diethylenetriamine, dipropylenetriamine, triethylenetetramine, and the like; hydrazines, such as hydrazine, N,N'-dimethylhydrazine, 1,6-hexamethylenebishydrazine, and the like; and dihydrazides, such as succinic acid dihydrazide, adipic acid dihydrazide, glutaric acid dihydrazide, sebacic acid dihydrazide, isophthalic acid dihydrazide, and the like. These may be used alone or in combination. Of these, polyamines including polyethyleneimine or polyallylamine are preferable from the viewpoint of readily obtaining molecules thereof having different molecular weights.

The polysaccharides are not particularly limited and may be appropriately selected depending on the intended purpose. Examples of the polysaccharides include chitosan, chitin, cellulose, agarose, carrageenan, heparin, hyaluronic acid, pectin, xyloglucan, glucomannan, starch, and glycogen. These may be used alone or in combination. Of these, chitosan is preferable because it contains an amino group.

The lower limit of the molar mass of the polymer is not particularly limited and may be appropriately selected depending on the intended purpose. From the viewpoint of the ability to adsorb biological particles, the lower limit thereof is preferably 500 g/mol or higher, more preferably 5000 g/mol or higher, still more preferably 10,000 g/mol or higher, and particularly preferably 60,000 g/mol or higher.

The upper limit of the molar mass of the polymer is not particularly limited and may be appropriately selected depending on the intended purpose. From the viewpoint of the ability to adsorb biological particles, the upper limit thereof is preferably 2,000,000 g/mol or lower, more preferably 1,000,000 g/mol or lower, still more preferably 500,000 g/mol or lower, and particularly preferably 200,000 g/mol or lower.

The polymer may be a branched-chain polymer or may be a linear polymer. From the viewpoint of the ability to adsorb biological particles, a branched-chain polymer is preferable.

(Method of producing structure)

The method of producing a structure includes connecting a water-insoluble fiber and a low-molecular-weight antibody to each other, and may further include other steps.

The water-insoluble fiber and the low-molecular-weight antibody are as described above.

A method of connecting the water-insoluble fiber and the low-molecular-weight antibody to each other is not particularly limited and may be appropriately selected depending on the intended purpose. Examples of the method include: a method in which the low-molecular-weight antibody is added to the water-insoluble fiber, followed by inversion mixing; and a method in which other elements are added to the water-insoluble fiber, followed by inversion mixing and the low-molecular-weight antibody is added to the resulting mixture, followed by inversion mixing.

The duration of the inversion mixing is not particularly limited and may be appropriately selected depending on the intended purpose. The duration thereof is preferably 1 hour or longer but 48 hours or shorter, more preferably 2 hours or longer but 48 hours or shorter, still more preferably 3 hours or longer but 24 hours or shorter, and particularly preferably 8 hours or longer but 24 hours or shorter.

A method of producing the structure including the spacer is not particularly limited and may be appropriately selected depending on the intended purpose. For example, the method includes connecting the water-insoluble fiber to one end of the spacer and connecting the low-molecular-weight antibody to the other end of the spacer.

The spacer, the water-insoluble fiber, and the low-molecular-weight antibody are as described above.

Here, the connecting the water-insoluble fiber to one end of the spacer and the connecting the low-molecular-weight antibody to the other end of the spacer can be performed in any order. From the viewpoint of readily controlling reaction for the connecting, preferably, the connecting the water-insoluble fiber to one end of the spacer is performed and then the connecting the low-molecular-weight antibody to the other end of the spacer is performed.

The method used to connect the water-insoluble fiber to one end of the spacer is not particularly limited and may be appropriately selected depending on the intended purpose. Examples thereof include a method in which a spacer solution is added to the water-insoluble fiber, followed by inversion mixing.

The duration of the inversion mixing is not particularly limited and may be appropriately selected depending on the intended purpose. The duration thereof is preferably 1 hour or longer but 48 hours or shorter, more preferably 2 hours or longer but 48 hours or shorter, still more preferably 3 hours or longer but 24 hours or shorter, and particularly preferably 8 hours or longer but 24 hours or shorter.

The method used to connect the low-molecular-weight antibody to the other end of the spacer is not particularly limited and may be appropriately selected depending on the intended purpose. Examples thereof include a method in which the low-molecular-weight antibody is added to the water-insoluble fiber, followed by inversion mixing.

The duration of the inversion mixing is not particularly limited and may be appropriately selected depending on the intended purpose. The duration thereof is preferably 1 hour or longer but 48 hours or shorter, more preferably 2 hours or longer but 48 hours or shorter, still more preferably 3 hours or longer but 24 hours or shorter, and particularly preferably 8 hours or longer but 24 hours or shorter.

The structure of the present invention can be processed depending on the intended purpose, and the shape thereof is not particularly limited. The structure can have a shape selected from, for example, a flat membrane shape, a hollow fiber shape, a pleat shape, a roll shape, a spiral shape, and a tubular shape. These processed structures may be used individually or may be stacked. Alternatively, the processed structures may be connected in series or parallel when used.

The shape of a device into which the structure is to be charged is not particularly limited and can be selected from, for example, a disc shape, a cylindrical shape, and a plate shape. From the viewpoint of realizing uniform liquid passage, a disc shape and a cylindrical shape are preferable.

The structure after processing can be used as, for example, a column for purifying biological particles, and a filter and a mask for adsorbing and removing biological particles. As a filter, the structure can be used as, for example, a filter for removing viruses in the production step for biopharmaceuticals such as blood preparations or the like, and a filter such as a HEPA (High Efficiency Particulate Air) filter or the like.

(Adsorbent)

The adsorbent includes the above structure and may further include other components.

The adsorption target of the adsorbent is as described above for the adsorption target of the low-molecular-weight antibody.

(Method of purifying biological particles)

The method of purifying biological particles includes a step of separation by an affinity chromatography purification method using the above structure or the above adsorbent, and may further include other steps.

The purification target of the method of purifying biological particles is as described above for the adsorption target of the low-molecular-weight antibody.

The purification is not particularly limited and may be appropriately selected depending on the intended purpose.

Examples of suitable purification include purification using a column into which the above structure or the above adsorbent is charged.

The material of the column is not particularly limited and may be appropriately selected depending on the intended purpose. Examples of the material include glass, resins such as polypropylene, acrylic, and the like, and metals such as stainless steel and the like.

Basically, the method of purifying biological particles can be achieved according to the same procedure as in a generally known purification method using an affinity column containing a protein ligand (examples of which include Protein A Column that is a commercially available product; Journal of Chromatography A 2007 1160:44-55).

That is, a buffer containing the target biological particles is adjusted to be neutral, and then the resulting solution is passed through an affinity column charged with the structure or adsorbent of the present invention, to allow the target biological particles to adsorb thereto.

Next, an appropriate amount of a pure buffer is passed through the affinity column, to wash the interior of the column. At this time, the desired biological particles are still adsorbed to the structure or adsorbent of the present invention in the column.

Next, an acidic or basic buffer adjusted to a suitable pH (this buffer may contain a substance that promotes dissociation from the structure or the adsorbent) is passed through the column to allow the desired biological particles to elute, and a high level of purification is achieved.

The structure or adsorbent of the present invention can be reused through washing by passage of an appropriate pure buffer that is such strongly acidic or strongly alkaline that the ligand (the low-molecular-weight antibody) and the base material of the carrier (the water-insoluble fiber) do not completely lose the functions thereof (this buffer may be a solution containing an appropriate denaturing agent or an organic solvent).

-Other steps-

The other steps are not particularly limited and may be appropriately selected depending on the intended purpose. Examples of the other steps include other purification steps such as an ion-exchange chromatography purification step, an ultracentrifugation purification step, and the like before or after the step of separation by the affinity chromatography purification method.

The ion-exchange chromatography purification step is a step of separating a mixture based on electrostatic interaction with an electrically charged ion-exchange ligand.

Also, the ultracentrifugation purification step is a step of centrifuging a mixture to separate the mixture for each substance having close molecular weights.

Compared with the other purification methods, the affinity chromatography purification method uses a ligand having an ability to specifically bind to the target biological particles, and thus advantageously, the affinity chromatography purification method more readily isolates the target biological particles with high purity from a mixture containing a large quantity of impurities such as a culture solution or the like.

-Elution rate of biological particles to flow-through (FT), recovery rate of biological particles in elution step, and residual rate of biological particles in washing step-

The elution rate of the biological particles to a flow-through (FT) in the method of purifying the biological particles is not particularly limited and may be appropriately selected depending on the intended purpose. The elution rate thereof is preferably lower than 100%, more preferably 50% or lower, still more preferably 20% or lower, and particularly preferably 0%.

The recovery rate of the biological particles in the elution step in the method of purifying the biological particles is not particularly limited and may be appropriately selected depending on the intended purpose. The recovery rate thereof is preferably 5% or higher, more preferably 10% or higher, still more preferably 50% or higher, and particularly preferably 70% or higher.

The residual rate of the biological particles in the washing step in the method of purifying the biological particles is not particularly limited and may be appropriately selected depending on the intended purpose. The residual rate thereof is preferably 30% or lower, more preferably 20% or lower, still more preferably 10% or lower, and particularly preferably 5% or lower.

The elution rate of the biological particles to the flow-through (FT), the recovery rate of the biological particles in the elution step, and the residual rate of the biological particles in the washing step are measured by the following method.

An AAV pre-treatment liquid is purified through chromatography with a nonwoven carrier.

The following Liquids A to F are prepared, and are filtered through a 0.2-$\mu$m filter before use.

Liquid A: Tris-HCl (20 mmol/L), sodium chloride (500 mmol/L), pH 8.0
Liquid B: citric acid (100 mmol/L), sodium chloride (500 mmol/L), pH 2.1
Liquid C: NaOH (10 mmol/L)
Liquid D: phosphoric acid (120 mmol/L), acetic acid (167 mmol/L), benzyl alcohol (2.2%v/v)
Liquid E: 20% ethanol
Liquid F: a solution prepared by diluting the AAV pre-treatment liquid with Liquid A and adjusted to pH 8.0 (about $1 \times 10^{12}$ vg)

A column charged with the carrier is connected to AKTA Avant 25. The flow rate of this system is set to 0.2 mL/min. The column is washed with pure water and equilibrated with Liquid A. Next, Liquid F is passed therethrough to retain the AAV to the column carrier, followed by washing with Liquid A and eluting the AAV with Liquid B. After completion of elution, the column is washed with Liquid A, then Liquid C, then Liquid A, and then Liquid D.

The AAV amount contained in each fraction is measured through quantitative PCR (qPCR) using QuantStudio3 Real-Time PCR System (obtained from Thermo Fisher Scientific) to calculate, as the recovery rate, the AAV amount of the elution peak relative to the AAV amount contained in the total recovery liquid. Also, the AAV amount in the flow-through (FT) solution relative to the total AAV amount of Liquid F passed through is calculated as the elution rate, and the AAV amount in the alkaline washing step relative to the AAV amount contained in the total recovery liquid is calculated as the residual rate.

Examples

The present invention will be described below by way of Examples. However, the present invention should not be construed as being limited to these Examples.

<Production Example 1: Production of water-insoluble fiber (nonwoven fabric 1)>

Using polypropylene as a material, a nonwoven fabric having an average fiber diameter of 1.19 $\mu$m, an areal weight of 30 g/m$^2$, and a thickness of 0.17 mm was produced by the melt-blow method. A radical-polymerizable compound solution was prepared by mixing GMA/POLYSOBATE 20/water so as to be a proportion by weight % of 10/2/88, followed by removal of dissolved oxygen through passage of nitrogen gas. In a nitrogen gas atmosphere, the produced nonwoven fabric was irradiated with electron beams at room temperature under conditions that the acceleration voltage was to be 250 kV and the irradiation dose was to be 50 kGy. The nonwoven fabric after the irradiation with electron beams was immersed in the radical-polymerizable compound solution, and was heated in a reaction tank at 50°C for 40 minutes to promote the polymerization reaction. After that, the nonwoven fabric was washed with water of normal temperature, followed by drying, to produce a water-insoluble fiber having a graft rate of 50% (nonwoven fabric 1).

As described below, the average fiber diameter and the average pore diameter of the nonwoven fabric were measured, and the nonwoven fabrics before and after the graft polymerization are summarized in Table 1. The results after the graft polymerization were also presented in Table 2.

The average fiber diameter of the nonwoven fabric was determined through observation under a scanning electron microscope. From the obtained electron microscope image, 100 or more fibers were randomly selected, and the diameters of the fibers were measured. The number average value of the 100 or more measured values was defined as the average fiber diameter. Also, the graft rate is a value calculated as follows from the measured weight of the nonwoven fabric before the graft reaction (W1) and the measured weight of the nonwoven fabric after the graft reaction (W2):

$$\texttt{Graft rate} = [(W2-W1)/W1] \times 100 \; (\%).$$

For the average pore diameter of the nonwoven fabric, the mean flow pore size measured with a perm porometer (obtained from Porous Materials Inc. (PMI), Capillary Flow Porometer CFP-1200AEXLCSHJ) was defined as the average pore diameter. For measurement and calculation of the average pore diameter, measurement software (Autofilling Perm-Porometer v6.73.90) and analysis software (CAPREP v6.71.51) were used.

The nonwoven fabric to be evaluated was punched out into a circle having a diameter of 26 mm, followed by immersion in Galwick (registered trademark in the US) penetration liquid (obtained from PMI, with a surface tension of 15.9 Dynes/cm). The resulting product was placed in a desiccator, from which air was removed by reducing the pressure with a vacuum pump for about 1 minute, and was charged with the penetration liquid. This nonwoven fabric was set on a sample stage (Φ26) attached to the device. The flow rate of air to the sample impregnated with the penetration liquid was gradually increased to 200000 cc/m. After that, the flow rate of air was gradually decreased until the pressure would reach 0 kPa in a dry state. The pressure in this process was plotted, and the average flow rate diameter (average pore diameter) was calculated by the bubble point method. Conditions for the measurement are presented below.

Maxflow 200000 (cc/m)
Bublflow 2.00 (cc/m)
F/PT 200 (oldbubltime)
Minbpress 0 (kPa)
Zerotime 1.0 (sec)
V2incr 5 (cts*3)
Preginc 0.3
Pulsedelay 2
Maxpres 1378.9466 (kPa)
Pulsewidth 0.2 (sec)
Mineqtime 30 (sec)
Presslew 10 (cts*3)
Flowslew 50 (cts*3)
Equiter 5 (0.1 sec)
Aveiter 20 (0.1 sec)
Maxpdif 0.69 (kPa)
Maxfdif 50 (cc/m)

Table 1

|  |  | Original fabric | After graft polymerization |
|---|---|---|---|
| Avg. fiber diameter | ($\mu$m) | 1.2 | 1.2 (unchanged) |
| Avg. pore diameter | ($\mu$m) | 3.3 | 3.4 (unchanged) |
| Areal weight | (g/m$^2$) | 30 | 45 |
| Thickness | (mm) | 0.17 | 0.2 |

Table 2

|  |  | Nonwoven fabric 1 | Nonwoven fabric 2 | Nonwoven fabric 3 | Nonwoven fabric 4 | Nonwoven fabric 5 | Nonwoven fabric 6 |
|---|---|---|---|---|---|---|---|
| Avg. fiber diameter | ($\mu$m) | 1.2 | 0.6 | 0.6 | 0.6 | 3.0 | 10 |
| Avg. pore diameter | ($\mu$m) | 3.4 | 3.7 | 3.5 | 3.5 | 10.9 | 32.8 |
| Areal weight | (g/m$^2$) | 45 | 26 | 18 | 17 | 45 | 50 |
| Thickness | (mm) | 0.2 | 0.17 | 0.11 | 0.13 | 0.28 | 0.26 |

<Production Example 2: Production of water-insoluble fiber (nonwoven fabric 2)>

Using polypropylene as a material, a nonwoven fabric having an average fiber diameter of 0.56 $\mu$m, an areal weight of 15 g/m$^2$, and a thickness of 0.11 mm was produced. Using a radical-polymerizable compound solution prepared by mixing GMA/POLYSOBATE 20/water so as to be a proportion by weight % of 8/2/90, graft polymerization by electron beams was performed in the same manner as in Production Example 1, to produce a water-insoluble fiber having a graft rate of 70% (nonwoven fabric 2).

In the same manner as in Production Example 1, the average fiber diameter and the average pore diameter of the nonwoven fabric were measured and were presented in Table 2.

<Production Example 3: Production of water-insoluble fiber (nonwoven fabric 3)>

Using polypropylene as a material, a nonwoven fabric having an average fiber diameter of 0.56 $\mu$m, an areal weight of 15 g/m$^2$, and a thickness of 0.11 mm was produced. Using a radical-polymerizable compound solution prepared by mixing GMA/POLYSOBATE 20/water so as to be a proportion by weight % of 2.4/2/95.6, graft polymerization by electron beams was performed in the same manner as in Production Example 1, to produce a water-insoluble fiber having a graft rate of 20% (nonwoven fabric 3).
In the same manner as in Production Example 1, the average fiber diameter and the average pore diameter of the nonwoven fabric were measured and were presented in Table 2.

<Production Example 4: Production of water-insoluble fiber (nonwoven fabric 4)>

Using polypropylene as a material, a nonwoven fabric having an average fiber diameter of 0.56 $\mu$m, an areal weight of 15 g/m$^2$, and a thickness of 0.11 mm was produced. A radical-polymerizable compound solution was prepared by mixing acrylic acid (AA)/neoethanol so as to be a proportion by weight % of 20/80, followed by removal of dissolved oxygen through passage of nitrogen gas. In a nitrogen gas atmosphere, the produced nonwoven fabric was irradiated with electron beams at room temperature under conditions that the acceleration voltage was to be 200 kV and the irradiation dose was to be 10 kGy. The nonwoven fabric after the irradiation with electron beams was immersed in the radical-polymerizable compound solution, and the resulting product was heated in a reaction tank at 50°C for 30 minutes to promote the polymerization reaction. After that, the nonwoven fabric was washed with water of normal temperature, followed by drying, to produce a water-insoluble fiber having a graft rate of 12% (nonwoven fabric 4).
In the same manner as in Production Example 1, the average fiber diameter and the average pore diameter of the nonwoven fabric were measured and were presented in Table 2.

<Production Example 5: Production of water-insoluble fiber (nonwoven fabric 5)>

Using polypropylene as a material, a nonwoven fabric having an average fiber diameter of 3 $\mu$m, an areal weight of 30 g/m$^2$, and a thickness of 0.28 mm was produced by the melt-blow method. Using a radical-polymerizable compound solution prepared by mixing GMA/POLYSOBATE 20/water so as to be a proportion by weight % of 10/2/88, graft polymerization by electron beams was performed in the same manner as in Production Example 1, to produce a water-insoluble fiber having a graft rate of 50% (nonwoven fabric 5).
In the same manner as in Production Example 1, the average fiber diameter and the average pore diameter of the nonwoven fabric were measured and were presented in Table 2.

<Water-insoluble fiber (nonwoven fabric 6)>

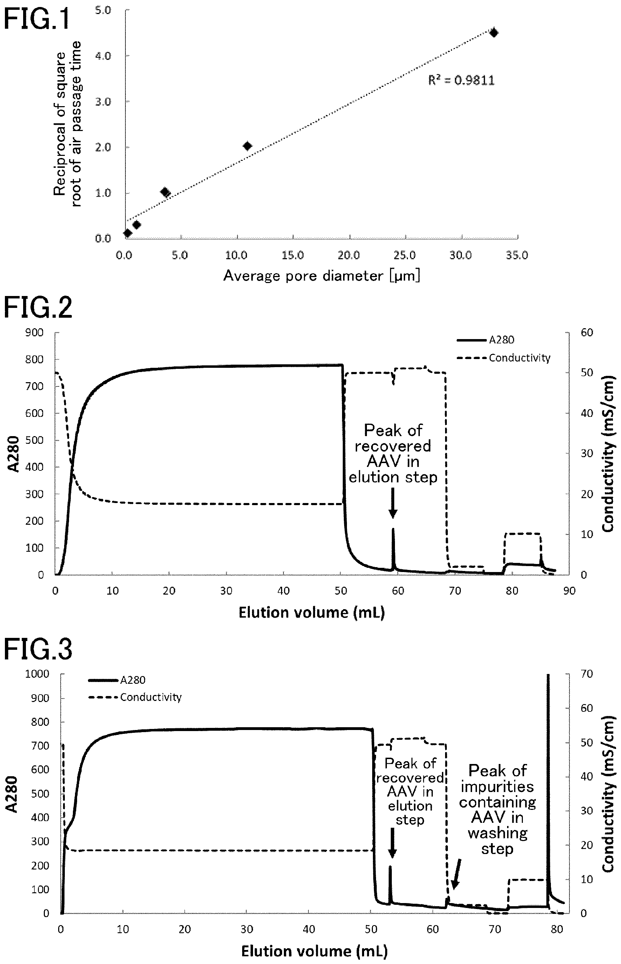

In the same manner as in Production Example 1, the average fiber diameter and the average pore diameter of a cellulose nonwoven fabric (hereinafter referred to as nonwoven fabric 6, 505SWJD, obtained from FUTAMURA CHEMICAL CO., LTD.) were measured and were presented in Table 2.
Nonwoven fabrics 2, 3, and 5 produced in Production Examples 2, 3, and 5 and nonwoven fabric 6 were measured for Gurley permeability with a Gurley type densometer (obtained from TOYOSEIKI).
The Gurley permeability was measured according to JIS L1096:2010 (8.26.2 B method (Gurley type method)). In this method, the water-insoluble fiber was set on a circular air-passage surface having an area of 642 mm$^2$. A cylinder having a height of 254 mm, an outer diameter of 76.2 mm, an inner diameter of 74 mm, and a mass of 567 g was used to pass 300 mL of air through each of the nonwoven fabrics. The time required for passage of the total amount of air of 300 mL (air passage time: sec) was measured. At this time, for nonwoven fabric 2, 3, or 5, the Gurley permeability was measured for each level three times when 5 sheets, 10 sheets, or 15 sheets were stacked on top of one another, and for nonwoven fabric 6, the Gurley permeability was measured three times for each level when 5 sheets, 10 sheets, 15 sheets, or 30 sheets were stacked on top of one another, and the average value of the Gurley permeabilities per sheet was calculated. The results were presented in Table 3.
The air passage time is believed to be inversely proportional to the area of the pores; i.e., the square of the average pore diameter. Then, the reciprocal of a square root of the air passage time was taken, and a correlation thereof to the average pore diameter calculated with a perm porometer was compared. The results were presented in Table 3 and FIG. 1.

Table 3

| | Average pore diameter [$\mu$m] | Time required for passage of air of 300 mL [s] | Square root of passage time | Reciprocal of square root of passage time |
|---|---|---|---|---|
| Nonwoven fabric 2 | 3.7 | 1.01 | 1.004 | 0.996 |
| Nonwoven fabric 3 | 3.5 | 0.94 | 0.970 | 1.031 |
| Nonwoven fabric 5 | 10.9 | 0.24 | 0.491 | 2.035 |
| Nonwoven fabric 6 | 32.8 | 0.05 | 0.222 | 4.506 |
| Membrane 1 | 0.2 | 60.67 | 7.789 | 0.128 |
| Membrane 3 | 1.0 | 9.84 | 3.137 | 0.319 |

As base materials having smaller pore dimeters, a regenerated cellulose membrane having an average pore diameter of 0.2 $\mu$m (obtained from cytiva, product code: 10410314, hereinafter referred to as membrane 1), a regenerated cellulose membrane having an average pore diameter of 0.45 $\mu$m (obtained from cytiva, product code: 10410214, hereinafter referred to as membrane 2), and a regenerated cellulose membrane having an average pore diameter of 1 $\mu$m (obtained from cytiva, product code: 10410014, hereinafter referred to as membrane 3) were provided. Of these, membrane 1 and membrane 3, each commercially available as a membrane having an enough size for measurement of air permeability, were measured for permeability in the same manner as in the nonwoven fabric, and a correlation thereof to the average pore diameter was compared. At this time, for both membrane 1 and membrane 3, the air permeability was measured three times for each level when 1 sheet, 3 sheets, or 5 sheets were stacked on top of one another, and the average value of the air permeabilities per sheet was calculated. The results were presented in Table 3 and FIG. 1.

From the results of Table 3 and FIG. 1, the average pore diameter of the nonwoven fabric and the average pore diameter of the membrane, which were measured with a perm porometer, is correlated well to the reciprocal of the square root of the air passage time, confirming that the correlation is not dependent on the kind and material of the base material.

<Production Example 6: Production of disc>

A punch was used to punch out each of nonwoven fabrics 1 to 5 produced in Production Examples 1 to 5 and nonwoven fabric 6 into a circle having a diameter of 5 mm, to produce a water-insoluble fiber disc (nonwoven disc) to be charged to a column.

In the same manner, a regenerated cellulose membrane having an average pore diameter of 0.2 $\mu$m (obtained from cytiva, product code: 10410314, membrane 1), a regenerated cellulose membrane having an average pore diameter of 0.45 $\mu$m (obtained from cytiva, product code: 10410214, membrane 2), and a regenerated cellulose membrane having an average pore diameter of 1 $\mu$m (obtained from cytiva, product code: 10410014, membrane 3) were each punched out into a circle having a diameter of 5 mm, to produce a base material disc (membrane disc) to be charged to a column.

<Production Example 7: Preparation of anti-AAV-scFv antibody>

Based on the heavy chain sequence (PDB:3J1S_H) and the light chain sequence (PDB:3J1S_L) of an antibody that binds to AAV2, which are registered in a protein databank, a single chain antibody that binds to AAV2 as set forth in SEQ ID NO: 1 in the Sequence Listing (hereinafter referred to as an anti-AAV-scFv antibody) was designed.

A gene for expression in *Escherichia coli* (SEQ ID NO: 2) in which a signal sequence (pelB) is added to this anti-AAV-scFv antibody was designed, and gene synthesized. A plasmid in which the gene synthesized sequence is introduced to an NcoI site and an NotI of a pET-22b(+) (obtained from Merck) vector was prepared by outsourcing (GenScript).

*E. coli* BL21 (DE3) (obtained from Merck) was transformed with the gene synthesized plasmid, followed by culturing in a Sakaguchi flask with Magic Media (obtained from Thermofisher) containing kanamycin, to produce an anti-AAV-scFv antibody.

The obtained culture solution was subjected to affinity purification using Protein L Carrier KANEKA KanCap L (obtained from KANEKA CORPORATION), and the buffer was changed through a centrifugal ultrafiltration membrane, to produce a purified product of the anti-AAV-scFv antibody.

<Production Example 8: Preparation 1 of anti-AAV-VHH antibody>

Based on the amino acid sequence of the anti-AAV-VHH antibody described in International Publication No. WO2018/104528 (SEQ ID NO: 59 in the Sequence Listing of International Publication No. WO2018/104528), an anti-AAV-VHH antibody for expression in *Escherichia coli* was designed (SEQ ID NO: 3).

A gene for expression in *Escherichia coli* (SEQ ID NO: 4) in which a signal sequence (pelB) is added to this anti-AAV-VHH antibody was designed, and gene synthesized. A plasmid in which the gene synthesized sequence is introduced to an XbaI site and a Bpu1102I site of a pET-28b(+) (obtained from Merck) vector was prepared by outsourcing (GenScript).

*E. coli* BL21 (DE3) (obtained from Merck) was transformed with the gene synthesized plasmid, followed by culturing in a Sakaguchi flask with Magic Media (obtained from Thermofisher) containing kanamycin, to produce an anti-AAV-VHH antibody.

The obtained culture solution was purified using cation exchange carrier Cellufine MAX S (obtained from JNC), and the buffer was changed through a centrifugal ultrafiltration membrane, to produce a purified product of the anti-AAV-VHH antibody (hereinafter referred to as VHH1).

<Production Example 9: Preparation 2 of anti-AAV-VHH antibody>

An immune experiment was performed by the method described in Example 6 of International Publication No. WO2020/067418. A group of antibody genes was obtained from blood of the immunized alpaca by the method described in Example 1 of JP-A No. 2015-119637, to prepare a phage library for an anti-AAV-VHH antibody. Screening of the anti-AAV-VHH antibody from the phage library was performed by the method of biopanning described in Example 1 of JP-A No. 2015-119637 using an empty particle of AAV as an antigen. The empty particle of AAV was prepared by the method described in Example 3 of International Publication No. WO2020/067418.

The phage-infected *E. coli* after the biopanning was serially diluted, and cultured in a 2YT agar medium (1.6% tryptone, 1.0% yeast extract, 2.0% agar) containing 100 $\mu$g/mL of ampicillin and 2% glucose. A single colony was cultured at 37°C overnight in 3 mL of a 2YT medium containing 100 $\mu$g/mL of ampicillin and 2% glucose. 30 $\mu$L of the culture solution cultured overnight was added to 5 mL of a 2YT medium containing 100 $\mu$g/mL of ampicillin, followed by culturing at 37°C and 300 rpm for 1 hour. After culturing, 3 mL of the culture solution was taken, and infected with a helper phage (M13KO7 (obtained from Thermo Fisher Scientific): 1.0×1013/mL) at MOI=5. After being left to stand still at 37°C for 30 minutes, the resulting product was shaken at 37°C and 300 rpm for 30 minutes. Kanamycin was added to the culture solution so as to have a concentration of 50 $\mu$g/mL, followed by culturing overnight at 37°C and 300 rpm. After culturing, the culture solution was centrifuged at 4°C and 4,000 rpm for 30 minutes, and the supernatant was filtered through a 0.2-$\mu$m filter (obtained from Sartorius), followed by removal of the *Escherichia coli,* to produce a phage in which anti-AAV-VHH antibodies were displayed.

Of these, anti-AAV-VHH antibodies having affinity to AAV2, AAV5, and AAV1 and 6 were referred to as VHH ligands 2, 3, and 4, respectively.

This phage solution was used as an antibody solution, and evaluated for binding activity to the AAV by the method described in International Publication No. WO2020/067418, to select a clone having a high binding activity. *E. coli* MV1184 (obtained from Takara Bio Inc.) was infected with the phage clone having a high binding activity, to breed *Escherichia coli* that would express the AAV-VHH antibodies in the periplasm thereof. This *Escherichia coli* was cultured at 37°C for 1 hour using a TB medium (1.2% tryptone, 2.4% yeast extract, 0.8% glycerol, 0.94% dipotassium hydrogenphosphate, 0.22% potassium dihydrogenphosphate) containing 100 $\mu$g/mL of ampicillin. After that, isopropyl-$\beta$-thiogalactopyranoside was added to the culture so as to have a final concentration of 1 mM, followed by culturing at 37°C overnight.

The anti-AAV-VHH antibodies were purified from the obtained VHH-expressing culture solution through cation exchange chromatography using AKTA avant 25 (obtained from GE Healthcare). A sample was applied to 100 mL of Cellufine MAX S-r (obtained from JNC) equilibrated with a 25 mM sodium phosphate buffer (pH 6.0) and was eluted with the buffer used for the equilibration and a linear gradient of a sodium phosphate buffer (pH 6.0) containing 1M NaCl. The purified anti-AAV-VHH antibodies were concentrated with centrifugal ultrafiltration unit OMEGA Membrane 1K (obtained from PALL), to produce purified VHH antibodies (VHH ligands 2, 3, and 4).

<Example 1: Production of carrier 1>

<Example 1-1: Immobilization of polyethyleneimine (PEI) spacer to nonwoven fabric 1>

A PEI solution (obtained from Alfa Aesar, Mw: 70,000, branched chain) was added to nonwoven fabric 1 obtained in Production Example 1, followed by inversion mixing at 37°C for 16 hours. After completion of reaction, the nonwoven fabric was transferred to a glass filter and washed with pure water, to produce nonwoven fabric 1 to which the PEI spacer was immobilized.

<Example 1-2: Functionalization of PEI spacer-immobilized nonwoven fabric>

4 mL of ultrapure water and 0.8 mL of a 2N sodium hydroxide solution were added to PEI spacer-immobilized nonwoven fabric 1 obtained in Example 1-1, followed by inversion mixing for 30 minutes. 4 mL of 1,4-butanediol diglycidyl ether (obtained from Nagase ChemteX Corporation) was added to the mixture, followed by inversion mixing at 37°C for 4 hours. After completion of reaction, the nonwoven fabric was transferred to a glass filter and washed with pure water, to produce epoxidized nonwoven fabric 1.

<Example 1-3: Immobilization of scFv ligand 1 to epoxidized nonwoven fabric 1>

A 0.2 mol/L carbonate buffer (pH 10.0) containing 0.5 mol/L NaCl was added to epoxidized nonwoven fabric 1 obtained in Example 1-2. To the resulting product, an amount equivalent to 1.7 mg of the purified scFv antibody (scFv ligand 1) produced in Production Example 7 was added so that the liquid amount was to be 5 mL. After inversion mixing at 37°C for 30 minutes, 0.497 g of sodium sulfate anhydrate was slowly added and dissolved, followed by further inversion mixing at 37°C for 16 hours. After completion of reaction, the nonwoven fabric was transferred to a glass filter and washed with the above carbonate buffer. The filtrate obtained this time was recovered, and the ligand density of the scFv ligand immobilized to the nonwoven fabric was calculated by measuring the absorbance thereof. The calculated ligand density was 0.4 mg/mL-column (Table 4). Subsequently, in order to seal the epoxy group remaining in the nonwoven fabric after washing, a 0.2 mol/L phosphate buffer (pH 8.0) containing 0.1 mol/L NaCl and 10% v/v thioglycerol was added, followed by inversion mixing at 25°C for 16 hours. After completion of reaction, the nonwoven fabric was transferred to a glass filter and washed with pure water and 20% ethanol, to produce scFv1-immobilized nonwoven fabric 1 (carrier 1).

Table 4

| Name of carrier | Base material | Spacer | Ligand | Ligand density (mg/mL-column) | (mol/mL-column) | Experiment |
|---|---|---|---|---|---|---|
| Carrier 1 | Nonwoven fabric 1 | PE1700000 | scFv1 | 0.4 | 1.2E-08 | Example |
| Carrier 2 | Nonwoven fabric 1 | - | VHH1 | 2.0 | 1.3E-07 | Example |
| Carrier 3 | Beads 1 | - | VHH2 | 4.1 | 2.8E-07 | Comp. Ex. |
| Carrier 4 | Membrane 1 | - | VHH2 | 14.0 | 9.6E-07 | Comp. Ex. |
| Carrier 5 | Membrane 2 | - | VHH2 | 12.3 | 8.4E-07 | Comp. Ex. |
| Carrier 6 | Membrane 3 | - | VHH2 | 5.5 | 3.8E-07 | Comp. Ex. |
| Carrier 7 | Nonwoven fabric 1 | PE1700000 | VHH2 | 2.4 | 1.6E-07 | Example |
| Carrier 8 | Nonwoven fabric 2 | PE1700000 | VHH2 | 2.8 | 1.9E-07 | Example |
| Carrier 9 | Nonwoven fabric 3 | PE1700000 | VHH2 | 3.9 | 2.7E-07 | Example |
| Carrier 10 | Nonwoven fabric 4 | - | VHH2 | 2.9 | 2.0E-07 | Example |
| Carrier 11 | Nonwoven fabric 5 | PE1700000 | VHH2 | 1.2 | 8.2E-08 | Example |

(continued)

| Name of carrier | Base material | Spacer | Ligand | Ligand density (mg/mL-column) (mol/mL-column) | | Experiment |
|---|---|---|---|---|---|---|
| Carrier 12 | Nonwoven fabric 6 | - | VHH2 | 3.3 | 2.3E-07 | Example |
| Carrier 13 | Beads 1 | - | VHH3 | 1.8 | 1.2E-07 | Comp. Ex. |
| Carrier 14 | Nonwoven fabric 1 | PE1700000 | VHH3 | 1.0 | 6.3E-08 | Example |
| Carrier 15 | Nonwoven fabric 1 | PE1700000 | VHH4 | 2.8 | 1.9E-07 | Example |

<Example 2: Production of carrier 2>

A VHH1-immobilized nonwoven fabric 1 (carrier 2) was produced through the spacer immobilization, the functionalization, and the VHH1 immobilization, performed in the same manner as in Example 1, except that scFv ligand 1 was changed to VHH ligand 1 and that the buffer for immobilization of the ligand was changed to a 0.2 mol/L carbonate buffer (pH 10.0) containing 0.5 mol/L NaCl and 1.2 mol/L guanidine hydrochloride.
The ligand density was measured in the same manner as in Example 1, and the measured ligand density was presented in Table 4.

<Comparative Example 1: Production of carrier 3>

<Comparative Example 1-1: Functionalization of beads 1>

POROS (registered trademark) 50 OH Hydroxyl Activated Resin (hereinafter referred to as beads 1, obtained from Thermo Scientific, with a pore diameter of from 50 to 1000 nm (nominal value)) was weighed in an amount of 5 mL-gel, followed by introduction of an epoxy group in the same manner as in Example 1-2, to produce beads 1 (epoxidized beads 1).
The ligand density was measured in the same manner as in Example 1, and the measured ligand density was presented in Table 4.

<Comparative Example 1-2: Immobilization of VHH2 to epoxidized beads 1>

A 0.2 mol/L carbonate buffer (pH 10.0) containing 0.5 mol/L NaCl was added to epoxidized beads 1 obtained in Comparative Example 1-2. To the resulting product, an amount equivalent to 5 mg of the purified VHH antibody ligand 2 produced in Production Example 9 was added so that the liquid amount was to be 5 mL. After inversion mixing at 37°C for 30 minutes, 0.497 g of sodium sulfate anhydrate was slowly added and dissolved, followed by further inversion mixing at 37°C for 16 hours. After completion of reaction, the beads were transferred to a glass filter and washed with the above carbonate buffer. The filtrate obtained this time was recovered, and the ligand density of the VHH ligand was calculated by measuring the absorbance thereof. Subsequently, in order to seal the epoxy group remaining in the beads after washing, a 0.2 mol/L phosphate buffer (pH 8.0) containing 0.1 mol/L NaCl and 10% v/v thioglycerol was added, followed by inversion mixing at 25°C for 16 hours. After completion of reaction, the beads were transferred to a glass filter and washed with pure water and 20% ethanol, to produce VHH2-immobilized beads 1 (carrier 3).
The ligand density was measured in the same manner as in Example 1, and the measured ligand density was presented in Table 4.

<Comparative Example 2: Production of carrier 4>

A VHH2-immobilized membrane 1 (carrier 4) was produced through the functionalization and the VHH2 immobilization, performed in the same manner as in Comparative Example 1, except that beads 1 was changed to regenerated cellulose membrane RC58 (membrane 1).
The ligand density was measured in the same manner as in Example 1, and the measured ligand density was presented in Table 4.

<Comparative Example 3: Production of carrier 5>

A VHH2-immobilized membrane 2 (carrier 5) was produced through the functionalization and the VHH2 immobilization, performed in the same manner as in Comparative Example 1, except that beads 1 was changed to regenerated cellulose membrane RC55 (membrane 2).
The ligand density was measured in the same manner as in Example 1, and the measured ligand density was presented in Table 4.

<Comparative Example 4: Production of carrier 6>

A VHH2-immobilized membrane 3 (carrier 6) was produced through the functionalization and the VHH2 immobilization, performed in the same manner as in Comparative Example 1, except that beads 1 was changed to regenerated cellulose membrane RC60 (membrane 3).
The ligand density was measured in the same manner as in Example 1, and the measured ligand density was presented in Table 4.

<Example 3: Production of carrier 7>

A VHH2-immobilized nonwoven fabric 1 (carrier 7) was produced through the spacer immobilization, the functionalization, and the VHH2 immobilization, performed in the same manner as in Example 1, except that scFv ligand 1 was changed to VHH ligand 2.
The ligand density was measured in the same manner as in Example 1, and the measured ligand density was presented in Table 4.

<Example 4: Production of carrier 8>

A VHH2-immobilized nonwoven fabric 2 (carrier 8) was produced through the spacer immobilization, the functionalization, and the VHH2 immobilization, performed in the same manner as in Example 1, except that nonwoven fabric 1 was changed to nonwoven fabric 2.
The ligand density was measured in the same manner as in Example 1, and the measured ligand density was presented in Table 4.

<Example 5: Production of carrier 9>

A VHH2-immobilized nonwoven fabric 3 (carrier 9) was produced through the spacer immobilization, the functionalization, and the VHH2 immobilization, performed in the same manner as in Example 1, except that nonwoven fabric 1 was changed to nonwoven fabric 3.
The ligand density was measured in the same manner as in Example 1, and the measured ligand density was presented in Table 4.

<Example 6: Production of carrier 10>

A 20% aqueous ethanol solution was added to nonwoven fabric 4. To the resulting product, an amount equivalent to 5 mg of the purified VHH antibody ligand 2 produced in Production Example 9 and an amount equivalent to 0.2 mg of 4-(4,6-dimethoxy-1,3,5-triazin-2-yl)-4-methylmorpholinium chloride (obtained from FUJIFILM Wako Pure Chemical Corporation) were added so that the liquid amount was to be 5 mL, followed by inversion mixing at 37°C for 4 hours. After completion of reaction, the nonwoven fabric was transferred to a glass filter and washed with pure water, to produce carrier 10. The filtrate obtained this time was recovered, and the ligand density of the VHH ligand was calculated by measuring the absorbance thereof.
The ligand density was measured in the same manner as in Example 1, and the measured ligand density was presented in Table 4.

<Example 7: Production of carrier 11>

A VHH2-immobilized nonwoven fabric 5 (carrier 11) was produced through the spacer immobilization, the functionalization, and the VHH2 immobilization, performed in the same manner as in Example 1, except that nonwoven fabric 1 was changed to nonwoven fabric 5.
The ligand density was measured in the same manner as in Example 1, and the measured ligand density was presented in Table 4.

<Example 8: Production of carrier 12>

A VHH2-immobilized nonwoven fabric 6 (carrier 12) was produced through the functionalization and the VHH2 immobilization, performed in the same manner as in Example 6, except that nonwoven fabric 4 was changed to nonwoven fabric 6.
The ligand density was measured in the same manner as in Example 1, and the measured ligand density was presented in Table 4.

<Comparative Example 5: Production of carrier 13>

VHH3-immobilized beads 1 (carrier 13) were produced through the functionalization and the VHH3 immobilization, performed in the same manner as in Comparative Example 1, except that VHH ligand 2 was changed to VHH ligand 3.
The ligand density was measured in the same manner as in Example 1, and the measured ligand density was presented in Table 4.

<Example 9: Production of carrier 14>

A VHH3-immobilized nonwoven fabric 1 (carrier 14) was produced through the spacer immobilization, the functionalization, and the VHH3 immobilization, performed in the same manner as in Example 1, except that scFv ligand 1 was changed to VHH ligand 3.
The ligand density was measured in the same manner as in Example 1, and the measured ligand density was presented in Table 4.

<Example 10: Production of carrier 15>

A VHH4-immobilized nonwoven fabric 1 (carrier 15) was produced through the spacer immobilization, the functionalization, and the VHH4 immobilization, performed in the same manner as in Example 1, except that scFv ligand 1 was changed to VHH ligand 4.
The ligand density was measured in the same manner as in Example 1, and the measured ligand density was presented in Table 4.

<Production Example 10: Charging of nonwoven fabric carrier to column>

Sheets of each of carriers 7 to 12, 14, and 15 produced in Examples 3 to 10 were stacked one sheet by one sheet on top of one another, and charged to a Tricorn (registered trademark) 5/20 column by an amount equivalent to 0.2 mL-column.

<Production Example 11: Charging of membrane carrier to column>

Sheets of each of carriers 4 to 6 produced in Comparative Examples 2 to 4 were stacked one sheet by one sheet on top of one another, and charged to a Tricorn (registered trademark) 5/20 column by an amount equivalent to 0.2 mL-column.

<Production Example 12: Charging of beads carrier to column>

Carriers 3 and 13 produced in Comparative Examples 1 and 5 and POROS (registered trademark) CaptureSelect (registered trademark) AAVX (obtained from Thermo Scientific) were each weighed by 0.2 mL-gel on the wet volume basis, and charged to a Tricorn (registered trademark) 5/20 column (obtained from GE Healthcare).

<Production Example 13: Production of adeno-associated virus (AAV2) by animal cells, and preparation of AAV2 pre-treatment liquid>

A plasmid for production of AAV2 that expresses VENUS (GenBank: ACQ43955.1), a variant of fluorescent protein GFP, was produced using an AAV vector production kit ("AAVpro (registered trademark) Helper Free System", obtained from Takara Bio Inc.).

Cultured HEK293 cells were transfected with the produced plasmid using a transfection reagent ("Polyethylenimine MAX", obtained from Polysciences, MW: 40,000) to produce AAV. After completion of culturing, the cells were peeled off to recover a cell culture solution. This was centrifuged to remove the supernatant, to produce AAV-producing cells.

The AAV-producing cells obtained above were suspended in Dulbecco's phosphate buffered saline (obtained from Sigma-Aldrich, hereinafter abbreviated as "PBS") containing 0.1% Triton X-100, followed by stirring in ice for 20 minutes, to disrupt the cells. To the obtained cell disruption liquid, 7.5 v/v% 1M aqueous magnesium chloride solution and 0.1 v/v% 250 kU/mL KANEKA Endonuclease (obtained from KANEKA CORPORATION) were added. The resulting mixture was left to stand still at 37°C for 30 minutes, to decompose the cell-derived nucleic acid. After completion of reaction, a 15 v/v% 0.5M EDTA solution was added to the reaction liquid, and the mixture was clarified through a depth filter (obtained from Pall). The obtained clarified liquid was used as the AAV2 pre-treatment liquid.

<Production Example 14: Preparation of AAV2 crude liquid>

Referring to a non-patent literature (Journal of Virological Methods 2007 140:183-192), the AAV2 pre-treatment liquid obtained in Production Example 13 was purified through cation exchange chromatography.

POROS (registered trademark) 50HS (obtained from Thermo) was charged to Tricorn (registered trademark) 10/150 (obtained from GE Healthcare) to prepare a column for cation exchange purification.

The following Liquids A to F were prepared, and were filtered through a 0.2-$\mu$m filter before use.

Liquid A: NaPO4 (20 mmol/L), NaCl (100 mmol/L), pH 7.4
Liquid B: NaPO4 (20 mmol/L), NaCl (100 mmol/L), Sarkosyl (5 mmol/L), pH 7.4
Liquid C: NaPO4 (20 mmol/L), NaCl (370 mmol/L), pH 7.4
Liquid D: NaCl (1 mol/L)
Liquid E: NaOH (0.5 mol/L)
Liquid F: a solution prepared by diluting the AAV2 pre-treatment liquid with Liquid A and adjusted to pH 7.4

The above column was connected to AKTA Avant 25, and was washed with Liquid D and equilibrated with Liquid A. Next, Liquid F was passed therethrough to retain the AAV to the column carrier, followed by washing with Liquid A, then Liquid B, and then Liquid A. The AAV was eluted with Liquid C. After completion of elution, the column was washed with Liquid D and Liquid E.

The AAV crude was confirmed through SDS-PAGE for the degree of purification, and the AAV amount in the liquid was quantitated through quantitative PCR (qPCR).

<Production Example 15: Production of adeno-associated viruses (AAV1, 5, and 6) by animal cells, and preparation of AAV1, 5, and 6 pre-treatment liquids>

AAV1, 5, and 6 pre-treatment liquids were prepared in the same manner as in Production Example 13, except that the plasmid for production of AAV2 was changed to plasmids for production of AAV1, 5, and 6.

<Test Example 1: Recovery test 1 of AAV by nonwoven fabric carrier>

A small quantity of the AAV2 crude liquid prepared in Production Example 14 was added to three discs of each of carriers 1 and 7 produced in Examples 1 and 3, followed by inversion mixing for 16 hours, to adsorb AAV2 thereto.

The supernatant was removed, followed by addition of an eluent (citric acid (100 mmol/L), sodium chloride (500 mmol/L), pH 2.1), to elute the AAV2 from the carrier.

The AAV amount contained in the liquid was quantitated through quantitative PCR (qPCR) using QuantStudio3 Real-Time PCR System (obtained from Thermo Fisher Scientific). When the measured AAV amount was converted to an adsorption amount per volume, the adsorption amount was $2.7 \times 10^{11}$ vg/mL-column for carrier 1 and was $1.9 \times 10^{12}$ vg/mL-column for carrier 7.

<Test Example 2: Recovery test 2 of AAV by nonwoven fabric carrier>

The recovery test of the AAV relative to carrier 2 produced in Example 2 was performed in the same manner as in Test Example 1, except that the liquid to be added was changed from the AAV2 crude liquid to the AAV1 pre-treatment liquid produced in Production Example 15. The amount of the AAV1 adsorbed was $1.1 \times 10^{11}$ vg/mL-column.

<Test Example 3: Recovery of AAV2 using nonwoven fabric carrier>

Using the columns of carriers 7 and 10 produced in Production Example 10, the AAV2 crude liquid obtained in Production Example 14 was purified through affinity chromatography.

The following Liquids A to F were prepared, and were filtered through a 0.2-$\mu$m filter before use.

Liquid A: Tris-HCl (20 mmol/L), sodium chloride (500 mmol/L), pH 8.0
Liquid B: citric acid (100 mmol/L), sodium chloride (500 mmol/L), pH 2.1
Liquid C: NaOH (10 mmol/L)
Liquid D: phosphoric acid (120 mmol/L), acetic acid (167 mmol/L), benzyl alcohol (2.2%v/v)
Liquid E: 20% ethanol
Liquid F: a solution prepared by diluting the AAV2 crude liquid with Liquid A and adjusted to pH 8.0 (about $1 \times 10^{12}$ vg)

The column charged with carrier 7 or 10 produced in Production Example 10 was connected to AKTA Avant 25. The flow rate of this system was set to 0.2 mL/min. The column was washed with pure water and equilibrated with Liquid A. Next, Liquid F was passed therethrough to retain the AAV to the column carrier, followed by washing with Liquid A and eluting the AAV with Liquid B (elution step). After completion of elution, the column was washed with Liquid A, then Liquid C, then Liquid A, and then Liquid D (washing step). After washing, the column was substituted with Liquid E and chilled for storage.

The AAV amount contained in each fraction was quantitated through quantitative PCR (qPCR) using QuantStudio3 Real-Time PCR System (obtained from Thermo Fisher Scientific). Also, the rate of the leaked AAV amount to the flow-through (FT) to the recovered AAV amount in the elution step, and the rate of the residual AAV amount in the washing step to the recovered AAV amount in the elution step were calculated. The results were presented in Table 5.

Table 5

| Carrier | Base material | Leaked AAV to FT (vg) | Recovered AAV in elution step (vg) | Residual AAV in washing step (vg) | Ratio of leaked AAV to recovered AAV ($\delta$) | Ratio of residual AAV to recovered AAV ($\delta$) | Experiment |
|---|---|---|---|---|---|---|---|
| Capture Select AAVX | Beads | 5.97E+09 | 8.77E+11 | 1.49E+11 | 1 | 17 | Comp. Ex. |
| Carrier 3 | Beads | 7.60E+10 | 2.00E+12 | 2.72E+11 | 4 | 14 | Comp. Ex. |
| Carrier 4 | Membrane | 2.07E+10 | 5.74E+11 | 2.29E+11 | 4 | 40 | Comp. Ex. |
| Carrier 5 | Membrane | 2.85E+10 | 6. 31E+11 | 1.07E+11 | 5 | 17 | Comp. Ex. |
| Carrier 6 | Membrane | 1.27E+10 | 4.76E+11 | 6.27E+10 | 3 | 13 | Comp. Ex. |
| Carrier 7 | Nonwoven fabric | 4.63E+09 | 9.41E+11 | 6.14E+10 | 0 | 7 | Ex. |
| Carrier 10 | Nonwoven fabric | 1.73E+10 | 9.57E+11 | 4.30E+10 | 2 | 4 | Ex. |

<Test Example 4: Recovery of AAV2 using membrane carrier>

Using columns of carriers 4, 5, and 6 produced in Production Example 11, the AAV2 crude liquid obtained in Production Example 14 was purified through affinity chromatography. In the same manner as in Test Example 3, the AAV amount contained in each fraction was quantitated, and the rate of the leaked AAV amount and the rate of the residual AAV amount were calculated. The results were presented in Table 5.

<Test Example 5: Recovery of AAV2 using bead carrier>

Using the columns of POROS (registered trademark) CaptureSelect (registered trademark) AAVX and carrier 3 produced in Production Example 12, the AAV2 crude liquid obtained in Production Example 14 was purified through affinity chromatography. In the same manner as in Test Example 3, the AAV amount contained in each fraction was quantitated, and the rate of the leaked AAV amount and the rate of the residual AAV amount were calculated. The results were presented in Table 5.

From the results presented in Table 5, the rate of the residual AAV amount was lower in carriers 7 and 10 than in POROS (registered trademark) CaptureSelect (registered trademark) AAVX and carriers 3 to 6. These results indicate that it is preferable to use the water-insoluble fiber as the base material of the carrier in order to efficiently recover AAV.

<Test Example 6: Recovery of AAV2 using nonwoven fabric carrier under high-flow-rate conditions>

Using the columns of carriers 7, 8, 9, 11, and 12 produced in Production Example 10, the AAV2 crude liquid obtained in Production Example 14 was purified through affinity chromatography. In the same manner as in Test Example 3 except that the flow rate of chromatography was changed from 0.2 mL/min to 1.2 mL/min (retention time: 10 seconds) or 2.4 mL/min (retention time: 5 seconds), the AAV amount contained in each fraction was quantitated, and the rate of the leaked AAV amount and the rate of the residual AAV amount were calculated. The results were presented in Table 6.

Table 6

| Carrier | Base material | Retention time (sec) | Leaked AAV to FT (vg) | Recovered AAV in elution step (vg) | Residual AAV in washing step (vg) | Ratio of leaked AAV to recovered AAV (δ) | Ratio of residual AAV to recovered AAV (δ) | Experiment |
|---|---|---|---|---|---|---|---|---|
| Capture Select AAVX | Beads | 10 | 4.19E+10 | 8.66E+11 | 1.47E+11 | 5 | 17 | Comp. Ex. |
| Capture Select AAVX | Beads | 5 | 1.38E+11 | 7.42E+11 | 1.67E+11 | 19 | 22 | Comp. Ex. |
| Carrier 7 | Nonwoven fabric | 10 | 2.58E+10 | 1.11E+12 | 5.76E+10 | 2 | 5 | Ex. |
| Carrier 7 | Nonwoven fabric | 5 | 1.64E+10 | 9.26E+11 | 9.63E+10 | 2 | 10 | Ex. |
| Carrier 8 | Nonwoven fabric | 10 | 2.88E+10 | 1.23E+12 | 4.06E+10 | 2 | 3 | Ex. |
| Carrier 8 | Nonwoven fabric | 5 | 2.31E+09 | 1.05E+12 | 4.85E+10 | 2 | 5 | Ex. |
| Carrier 9 | Nonwoven fabric | 10 | 2.50E+10 | 1.36E+12 | 4.13E+10 | 2 | 3 | Ex. |
| Carrier 9 | Nonwoven fabric | 5 | 2.38E+10 | 1.04E+12 | 4.94E+10 | 2 | 5 | Ex. |
| Carrier 11 | Nonwoven fabric | 10 | 6.01E+08 | 8.12E+11 | 3.16E+10 | 0 | 4 | Ex. |
| Carrier 12 | Nonwoven fabric | 10 | 8.14E+11 | 2.13E+12 | 2.98E+10 | 38 | 1 | Ex. |

<Test Example 7: Recovery of AAV2 using bead carrier under high-flow-rate conditions>

Using the column of POROS (registered trademark) CaptureSelect (registered trademark) AAVX produced in Production Example 12, the AAV2 crude liquid obtained in Production Example 14 was purified through affinity chromatography. In the same manner as in Test Example 3 except that the flow rate of chromatography was changed from 0.2 mL/min to 1.2 mL/min (retention time: 10 seconds) or 2.4 mL/min (retention time: 5 seconds), the AAV amount contained in each fraction was quantitated, and the rate of the leaked AAV amount and the rate of the residual AAV amount were calculated. The results were presented in Table 6.

From the results of Table 6, nonwoven fabric carriers 7, 8, 9, and 11 were found to be that even if the flow rate is increased to make the retention time shorter than 1 minute, the rate of the AAV leaked to the FT is low, and also the rate of the residual AAV is low. Meanwhile, for carrier 12, the rate of the residual AAV is low, but the rate of the leaked AAV is high. Note that, for carrier 11, the amount of the leaked AAV to the FT is a value lower than those of the other carriers. This is however because the value was outside the lower-limit range of a calibration curve. The result of carrier 11 is expected to be comparable to the results of carriers 7, 8, and 9.

<Test Example 8: Purification of AAV2 by carrier 7>

Using the column of carrier 7 produced in Production Example 10, the AAV2 pre-treatment liquid obtained in Production Example 13 was purified through affinity chromatography.

The following Liquids A to F were prepared, and were filtered through a 0.2-$\mu$m filter before use.

Liquid A: Tris-HCl (20 mmol/L), sodium chloride (500 mmol/L), pH 8.0
Liquid B: citric acid (100 mmol/L), sodium chloride (500 mmol/L), pH 2.1
Liquid C: NaOH (10 mmol/L)
Liquid D: phosphoric acid (120 mmol/L), acetic acid (167 mmol/L), benzyl alcohol (2.2%v/v)
Liquid E: 20% ethanol
Liquid F: a solution prepared by diluting the AAV2 pre-treatment liquid with Liquid A and adjusted to pH 8.0 (about $1 \times 10^{12}$ vg)

The column charged with carrier 7 produced in Production Example 10 was connected to AKTA Avant 25. The flow rate of this system was set to 0.2 mL/min. The column was washed with pure water and equilibrated with Liquid A. Next, Liquid F was passed therethrough to retain the AAV to the column carrier, followed by washing with Liquid A and eluting the AAV with Liquid B. After completion of elution, the column was washed with Liquid A, then Liquid C, then Liquid A, and then Liquid D. After washing, the column was substituted with Liquid E and chilled for storage. A chromatogram during purification is illustrated in FIG. 2.

Also, the AAV amount contained in each fraction was quantitated through quantitative PCR (qPCR) using QuantStudio3 Real-Time PCR System (obtained from Thermo Fisher Scientific). Also, the rate of the residual AAV amount in the washing step to the recovered AAV amount in the elution step were calculated. The results were presented in Table 7.

Table 7

| | Base material | Recovered AAV in elution step (vg) | Residual AAV in washing step (vg) | Ratio of residual AAV to recovered AAV ($\delta$) | Experiment |
|---|---|---|---|---|---|
| Capture Select AAVX | Beads | 5.75E+11 | 2.80E+11 | 48.8 | Comparative Example |
| Carrier 7 | Nonwoven fabric | 1.57E+12 | 4.93E+10 | 3.1 | Example |

<Test Example 9: Purification of AAV2 by bead carrier>

The AAV pre-treatment liquid was purified through affinity chromatography in the same manner as in Test Example 8, except that the column of carrier 7 was changed to the column charged with POROS (registered trademark) CaptureSelect (registered trademark) AAVX produced in Production Example 12. The obtained chromatogram is illustrated in FIG. 3. In the same manner as in Test Example 8, the recovered AAV in the elution step and the residual AAV in the washing step were calculated. The results were presented in Table 7.

<Test Example 10: Purification of AAV5 by carrier 14>

Using the column of carrier 14 produced in Production Example 10, the AAV5 pre-treatment liquid obtained in Production Example 15 was purified through affinity chromatography.

The following Liquids A to F were prepared, and were filtered through a 0.2-$\mu$m filter before use.

Liquid A: Tris-HCl (20 mmol/L), sodium chloride (100 mmol/L), pH 8.0
Liquid B: citric acid (20 mmol/L), sodium chloride (100 mmol/L), pH 2.1
Liquid C: NaOH (10 mmol/L)
Liquid D: phosphoric acid (120 mmol/L), acetic acid (167 mmol/L), benzyl alcohol (2.2%v/v)
Liquid E: 20% ethanol
Liquid F: a solution prepared by diluting the AAV5 pre-treatment liquid with Liquid A and adjusted to pH 8.0 (about $1\times10^{12}$ vg)

The column charged with carrier 14 produced in Production Example 10 was connected to AKTA Avant 25. The flow rate of this system was set to 0.2 mL/min. The column was washed with pure water and equilibrated with Liquid A. Next, Liquid F was passed therethrough to retain the AAV to the column carrier, followed by washing with Liquid A and eluting the AAV with Liquid B. After completion of elution, the column was washed with Liquid A, then Liquid C, then Liquid A, and then Liquid D. After washing, the column was substituted with Liquid E and chilled for storage.

Also, the AAV amount contained in each fraction was quantitated through quantitative PCR (qPCR) using QuantStudio3 Real-Time PCR System (obtained from Thermo Fisher Scientific). Also, the rate of the residual AAV amount in the washing step to the recovered AAV amount in the elution step were calculated. The results were presented in Table 8.

Table 8

|  | Base material | Recovered AAV in elution step (vg) | Residual AAV in washing step (vg) | Ratio of residual AAV to recovered AAV ($\delta$) | Experiment |
|---|---|---|---|---|---|
| Carrier 13 | Beads | 9.91E+11 | 9.92E+10 | 10.0 | Comparative Example |
| Carrier 14 | Nonwoven fabric | 3.65E+11 | 5.74E+09 | 1.6 | Example |

<Test Example 11: Purification of AAV5 by carrier 13>

In the same manner as in Test Example 10 except that the column of carrier 14 was changed to a column charged with carrier 13 produced in Production Example 12, the AAV5 pre-treatment liquid was purified through affinity chromatography. In the same manner as in Test Example 10, the recovered AAV in the elution step and the residual AAV in the washing step were calculated. The results were presented in Table 8.

<Test Example 12: Purification of AAV1 and 6 by carrier 15>

Using the column of carrier 15 produced in Production Example 10, the AAV1 pre-treatment liquid and the AAV6 pre-treatment liquid obtained in Production Example 15 were purified through affinity chromatography. In the same manner as in Test Example 10 except that Liquid F was changed to the AAV1 pre-treatment liquid or the AAV6 pre-treatment liquid, the AAV1 and 6 were purified. The AAV amount contained in the elution step was $2.2\times10^{10}$ vg/mL-column for AAV1 and $4.4\times10^{10}$ vg/mL-column for AAV6.

From the results of Test Examples 8 to 12, the structure of the present application was found not to be limited to a serotype of AAV, and widely applicable.

From the results of FIGS. 1 and 2 and Tables 5 to 8, when using bead carriers such as POROS (registered trademark) CaptureSelect (registered trademark) AAVX, carrier 14, or the like, a certain amount of AAV was detected in the washing step after the elution step. Accordingly, it may be concluded that the bead carriers are materials having difficulty in sufficiently recovering AAV clogged inside the carrier.

Meanwhile, when using the structure of the present invention as the carrier, a single peak appeared in the elution step, and the amount of the residual AAV in the washing step after the elution step was considerably small. Accordingly, it may be concluded that the structure of the present invention is a material that can efficiently recover AAV without clogging thereof inside the carrier.

In view of the above, the structure of the present application is found to be a structure having a high biological particle recovery rate in the elution step and a low biological particle residual rate in the washing step.

<Test Example 13: Calculation of AAV-binding capacity of nonwoven carrier>

Using the column of carrier 7 produced in Production Example 10, the AAV crude liquid obtained in Production Example 14 was purified through affinity chromatography.

The following Liquids A to F were prepared, and were filtered through a 0.2-$\mu$m filter before use.

Liquid A: Tris-HCl (20 mmol/L), sodium chloride (500 mmol/L), pH 8.0
Liquid B: citric acid (100 mmol/L), sodium chloride (500 mmol/L), pH 2.1
Liquid C: NaOH (10 mmol/L)
Liquid D: phosphoric acid (120 mmol/L), acetic acid (167 mmol/L), benzyl alcohol (2.2%v/v)
Liquid E: 20% ethanol
Liquid F: a solution prepared by diluting the AAV crude liquid with Liquid A and adjusted to pH 8.0 (about $2\times10^{13}$ vg)

The column was connected to AKTA Avant 25. The flow rate of this system was set to 0.2 mL/min. The column was washed with pure water and equilibrated with Liquid A. Next, Liquid F was passed therethrough to retain the AAV to the column carrier, followed by washing with Liquid A and eluting the AAV with Liquid B. After completion of elution, the column was washed with Liquid A, then Liquid C, then Liquid A, and then Liquid D. After washing, the column was substituted with Liquid E and chilled for storage. The AAV amount contained in each fraction was quantitated through qPCR, to calculate the binding capacity from the AAV amount contained in the elution step. The results were presented in Table 9.

Table 9

| Carrier | AAV binding capacity (vg/mL-column) |
| --- | --- |
| Carrier 7 | $2.6\times10^{13}$ |
| Capto AVB | $>1\times10^{12}$ (nominal value) |
| CaptureSelect AAVX | $>1\times10^{13}$ (nominal value) |

From the results of Table 9, also in terms of virus adsorption, the structure of the present invention was found to be exhibit comparable performances to those of commercially available products. Note that, the AAV crude liquid that is prepared this time contains not only AAV containing virus genes but also a large quantity of capsid not containing any genes. Because the qPCR measurement quantifies only the AAV containing virus genes, the actual binding capacity is expected to be larger than the value presented in Table 9.

Aspects of the present invention are as follows, for example.

<1> A structure, including:

a water-insoluble fiber; and
a low-molecular-weight antibody connected to the water-insoluble fiber.

<2> The structure according to <1> above, wherein the water-insoluble fiber is a nonwoven fabric.
<3> The structure according to <2> above, wherein the nonwoven fabric includes polyolefin and a derivative thereof.
<4> The structure according to any one of <1> to <3> above, wherein the low-molecular-weight antibody is capable of adsorbing a virus, a virus-like particle, or a virus vector.
<5> The structure according to <4> above, wherein the virus, the virus-like particle, or the virus vector is an adeno-associated virus or a capsid of the adeno-associated virus, or a virus vector including an adeno-associated virus gene.
<6> The structure according to any one of <1> to <5> above, wherein the low-molecular-weight antibody lacks a CH2 domain and a CH3 domain.
<7> The structure according to any one of <1> to <6> above, wherein the low-molecular-weight antibody includes a heavy-chain variable region or a light-chain variable region of a whole antibody.
<8> The structure according to any one of <1> to <7> above, wherein the low-molecular-weight antibody includes a single domain antibody.

<9> The structure according to <8> above, wherein the single domain antibody includes a variable region of a heavy-chain antibody derived from a camelid.

<10> The structure according to any one of <1> to <9> above, wherein the low-molecular-weight antibody is connected via a spacer to the water-insoluble fiber.

<11> The structure according to <10> above, wherein the spacer includes an amino group, a hydroxy group, an epoxy group, or a carboxy group.

<12> The structure according to <10> or <11> above, wherein the spacer includes a polymer.

<13> A method of producing a structure, the method including:

connecting a water-insoluble fiber and a low-molecular-weight antibody to each other.

<14> An adsorbent, including:

the structure according to any one of <1> to <12> above.

<15> A method of purifying biological particles, the method including:

using the structure according to any one of <1> to <12> above or the adsorbent according to <14> above.

**Claims**

1. A structure, comprising:

   a water-insoluble fiber; and
   a low-molecular-weight antibody connected to the water-insoluble fiber.

2. The structure according to claim 1, wherein the water-insoluble fiber is a nonwoven fabric.

3. The structure according to claim 2, wherein the nonwoven fabric includes polyolefin and a derivative thereof.

4. The structure according to any one of claims 1 to 3, wherein the low-molecular-weight antibody is capable of adsorbing a virus, a virus-like particle, or a virus vector.

5. The structure according to claim 4, wherein the virus, the virus-like particle, or the virus vector is an adeno-associated virus or a capsid of the adeno-associated virus, or a virus vector including an adeno-associated virus gene.

6. The structure according to any one of claim 1 to 5, wherein the low-molecular-weight antibody lacks a CH2 domain and a CH3 domain.

7. The structure according to any one of claims 1 to 6, wherein the low-molecular-weight antibody includes a heavy-chain variable region or a light-chain variable region of a whole antibody.

8. The structure according to any one of claims 1 to 7, wherein the low-molecular-weight antibody includes a single domain antibody.

9. The structure according to claim 8, wherein the single domain antibody includes a variable region of a heavy-chain antibody derived from a camelid.

10. The structure according to any one of claims 1 to 9, wherein the low-molecular-weight antibody is connected via a spacer to the water-insoluble fiber.

11. The structure according to claim 10, wherein the spacer includes an amino group, a hydroxy group, an epoxy group, or a carboxy group.

12. The structure according to claim 10 or 11, wherein the spacer includes a polymer.

13. A method of producing a structure, the method comprising:
    connecting a water-insoluble fiber and a low-molecular-weight antibody to each other.

14. An adsorbent, comprising:
    the structure according to any one of claims 1 to 12.

**15.** A method of purifying biological particles, the method comprising:
using the structure according to any one of claims 1 to 12 or the adsorbent according to claim 14.

FIG.1

FIG.2

FIG.3

## INTERNATIONAL SEARCH REPORT

| International application No. |
| --- |
| PCT/JP2021/016772 |

**A. CLASSIFICATION OF SUBJECT MATTER**
C07K 17/08(2006.01)i; C12N 7/02(2006.01)i
FI: C07K17/08; C12N7/02

According to International Patent Classification (IPC) or to both national classification and IPC

**B. FIELDS SEARCHED**

Minimum documentation searched (classification system followed by classification symbols)
C07K17/08; C12N7/02

| Documentation searched other than minimum documentation to the extent that such documents are included in the fields searched | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2007-159874 A (ASAHI KASEI CORPORATION) 28 June 2007 (2007-06-28) example 1 | 1-3, 6-7, 10-15 |
| Y | | 1-15 |
| X | WO 03/101611 A1 (JAPAN ENVIROCHEMICALS, LTD.) 11 December 2003 (2003-12-11) claims 1, 3, 6, 10, | 1-3, 6-7, 10-15 |
| Y | example 4, page 60, lines 3-4, fig. 1 | 1-15 |
| Y | JP 2018-507707 A (INSTITUT NATIONAL DE LA SANTE ET DE LA RECHERCHE MEDICALE (INSERM)) 22 March 2018 (2018-03-22) claims 1, 2, 4 | 1-15 |

☒ Further documents are listed in the continuation of Box C.　☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 18 May 2021 (18.05.2021) | 25 May 2021 (25.05.2021) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
|---|
| PCT/JP2021/016772 |

C (Continuation).   DOCUMENTS CONSIDERED TO BE RELEVANT

| Category* | Citation of document, with indication, where appropriate, of the relevant passages | Relevant to claim No. |
|---|---|---|
| Y | 岡田尚巳，AAV ベクターの大量製造法と精製法，実験医学，01 February 2020, vol. 38, no. 2 (extra issue), pp. 90(232) to 95(237), ISBN978-4-7581-0384-8, in particular, p. 92(234), left column, line 5 from the bottom to the bottom line, table of p. 93(235), non-official translation (OKADA, Takashi, "Production method and purification method of AAV vectors", Experimental Medicine) | 8-9 |
| Y | 宮崎誠生ほか，2 ラクダ科動物由来天然起源シングルドメイン抗体 (VHH 抗体）の開発，次世代医薬開発に向けた抗体工学の最前線，普及版，株式会社シーエムシー出版， 2019, pp. 124-129, ISBN978-4-7813-1378-8, in particular, pp. 125-126, section "2.4 Excellent features of VHH antibody", non-official translation (MIYAZAKI, Nobuo et al., "2 Development of naturally occurring Camelids single-domain antibodies (VHH antibody) derived from animals", Frontier of Antibody Engineering for Next-generation Therapeutics, Popular Edition, CMC PUBLISHING CO., LTD.) | 8-9 |

Form PCT/ISA/210 (continuation of second sheet) (January 2015)

**INTERNATIONAL SEARCH REPORT**
Information on patent family members

International application No.

PCT/JP2021/016772

| Patent Documents referred in the Report | Publication Date | Patent Family | Publication Date |
|---|---|---|---|
| JP 2007-159874 A | 28 Jun. 2007 | (Family: none) | |
| WO 03/101611 A1 | 11 Dec. 2003 | US 2006/0216761 A1 claims 1, 3, 6, 10, example 4, paragraph [0229], fig. 1 EP 1514597 A1 | |
| JP 2018-507707 A | 22 Mar. 2018 | WO 2016/128408 A1 claims 1, 2, 4 US 2019/0382733 A1 EP 3054007 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0144301 A
- WO 2020067418 A
- JP 2015119637 A
- WO 2018104528 A

**Non-patent literature cited in the description**

- *Mol. Ther. Methods Clin. Dev.,* 2018, vol. 9, 33
- *Proc. Natl. Acad. Sci. U.S.A,* 1988, vol. 85, 5879
- *Journal of Chromatography A,* 2007, vol. 1160, 44-55
- *Journal of Virological Methods,* 2007, vol. 140, 183-192